# EUROPEAN PATENT APPLICATION

(11) **EP 4 534 108 A1**
(43) Date of publication of application: **09.04.2025**
(21) Application number: 23816007.1
(22) Date of filing: 29.05.2023
(51) Int. Cl.: A61K 47/69, A61K 9/08, A61K 9/19, A61K 31/7088, A61K 45/00, A61K 47/12, A61K 47/24, A61K 48/00, A61P 17/00, C12N 15/88

(54) **COMPOSITION CONTAINING ANIONIC LONG-CHAIN LIPID TO BE USED FOR ATTENUATING SKIN BARRIER FUNCTION, AND DISPERSION CONTAINING COMPLEX COMPRISING CATIONIC LONG-CHAIN LIPID, ANIONIC LONG-CHAIN LIPID AND DRUG DISPERSED THEREIN**

(30) Priority: 30.05.2022 JP 2022087623
(71) Applicant: Kyushu University, National University Corporation, Nishi-ku Fukuoka-shi Fukuoka 819-0395 (JP); Novigo Pharma, Inc., Fukuoka-shi, Fukuoka 819-0388 (JP)
(72) Inventor: TOYOFUKU Kiyohiro, Fukuoka-shi, Fukuoka 819-0395 (JP); GOTO Masahiro, Fukuoka-shi, Fukuoka 819-0395 (JP); KITAOKA Momoko, Fukuoka-shi, Fukuoka 819-0388 (JP)
(74) Representative: Klöckner, Christoph
(86) International application number: PCT/JP2023/019898
(87) International publication number: WO 2023/234250

(57) **Abstract**

The present invention provides a composition for use in attenuating skin barrier, the composition containing an anionic long-chain lipid (in particular, a long-chain fatty acid). The present invention provides a dispersion in which complexes (e.g., solid complexes) each containing a cationic long-chain lipid, an anionic long-chain lipid, and a drug are dispersed.

## Description

### Technical Field

The present invention relates to a composition for use in attenuating skin barrier, the composition containing an anionic long-chain lipid (in particular, a long-chain fatty acid). The present invention relates to a dispersion in which solid complexes each containing a cationic long-chain lipid, an anionic long-chain lipid, and a drug are dispersed.

### Background Art

Penetration of hydrophilic molecules of more than 800 Da through the skin is extremely low because of the barrier function of the horny layer. The barrier function of the horny layer limits development of formulations for transdermal administration (percutaneous absorption agents). As countermeasures to this, attempts have been made to develop a dispersion formulation in which solid complexes each containing a hydrophilic pharmaceutical agent of more than 800 Da covered with a surfactant are dispersed in an oil phase (Patent Literatures 1 to 4). These allow various molecules including small molecules and proteins to be transdermally administered to an individual, and thus have even enabled transfer of hydrophilic molecules of more than 800 Da into blood through transdermal administration (Patent Literatures 1 to 4).

A technique for forming solid complexes with use of an ionic liquid has been disclosed (Patent Literature 5). Patent Literature 5 proposes a novel cationic lipid to adapt the ionic liquid to living bodies.

### Citation List

### Patent Literature

Patent Literature 1: Japanese Patent No. 4426749
Patent Literature 2: Japanese Patent No. 4843494
Patent Literature 3: Japanese Patent No. 5752343
Patent Literature 4: WO 2021/085491A
Patent Literature 5: WO 2021/192861A

### Summary of Invention

The present disclosure provides a composition containing complexes (particles) each containing a cation (preferably a cationic surfactant, more preferably a cationic lipid), an anion (preferably an anionic surfactant, more preferably a fatty acid, still more preferably a long-chain fatty acid), and a nucleic acid.

The present inventors have found that anionic long-chain lipids attenuate skin barrier function; and that because of this, anionic long-chain lipids are applicable to enhancement of the penetration of drugs into skin. In addition, the present inventors have conceived a composition containing solid complexes and an oil phase, wherein the solid complexes contain a cation and an anion, in particular, a cationic lipid and an anionic lipid, and a pharmaceutical agent (e.g., a nucleic acid), and are dispersed in the oil phase.

The present invention provides the followings.
(1) A composition for use in formation of a complex with a pharmaceutical ingredient, the composition containing a mixture of a cation and an anion (preferably, the composition consists of a cation and an anion, or consists of a cation, an anion, and a solvent).
(2) The composition according to (1) for transdermal administration, wherein the cation is a cationic lipid (e.g., EDMPC), and the anion is a fatty acid (e.g., an unsaturated fatty acid, preferably a long-chain unsaturated fatty acid, more preferably linoleic acid and oleic acid).
(3) The composition according to (1) or (2), containing no pharmaceutical active ingredient.
(4) The composition according to (1) or (2), further containing a pharmaceutical active ingredient to be transdermally administered.
(5) A composition (preferably a composition for transdermal administration) containing:
   a population of solid complexes containing a cation, an anion, and a nucleic acid; and
   an oil phase, wherein
   the population of the solid complexes is dispersed in the oil phase.
(6) The composition according to (5), wherein the population of the solid complexes includes a solid complex of about 5 nm to about 50 nm.
(7) The composition according to (6), wherein the population of the solid complexes includes a structure including a plurality of aggregates of solid complexes of about 5 nm to about 50 nm.
(8) The composition according to any one of (5) to (7), for use in transdermal administration of the nucleic acid.
(9) The composition according to any one of (5) to (8), wherein the cation is a cationic lipid, and the anion is an anionic lipid.
(10) The composition according to any one (5) to (9), wherein the cation and the anion are contained at an electric charge ratio of 1:0.8 to 1:1.2.
(11) The composition according to any one of (5) to (10), wherein the cation and the anion are capable of forming an ionic liquid at least when the cation and the anion are mixed together at an electric charge ratio of 1:1 in a composition consisting only of the cation and the anion.
(12) An aqueous composition containing a complex containing a cation, an anion, and a nucleic acid, wherein
   the composition allows transfection with the nucleic acid from an exterior of a cell to an interior of the cell and thereby enables production of a viable cell containing the nucleic acid. therein
(13) A method for producing the composition according to any one of (5) to (11), the method including:
   mixing an aqueous solution containing a nucleic acid and a lower alcohol solution containing a mixture containing a cation and an anion to obtain a complex containing the nucleic acid and the cation and anion.
(14) The method according to (13), further including freeze-drying the complex provided.
(15) The method according to (12) or (13), including dispersing a freeze-dried complex in an oil phase.
(16) The composition according to (12), for use in transfection with a nucleic acid from an exterior of a cell to an interior of the cell.
(17) A composition for use in attenuating skin barrier (or enhancing skin permeation) in a subject, the composition containing an anionic lipid.
(18) The composition according to (17), wherein the anionic lipid is a fatty acid.
(19) The composition according to (17) or (18), wherein the anionic lipid is a long-chain fatty acid.
(20) The composition according to any one of (17) to (19), wherein the anionic lipid is a long-chain unsaturated fatty acid.
(21) The composition according to any one of the above invention, wherein the cationic lipid and the anionic lipid are a cationic fatty acid and an anionic fatty acid, respectively.
(22) A composition, wherein a cationic lipid and an anionic lipid are capable of forming an ionic liquid at least in a composition containing only the cationic lipid and the anionic lipid at an electric charge ratio of 1:1.
(23) The composition according to (21), wherein the cationic fatty acid and the anionic fatty acid are capable of forming an ionic liquid at least when the cationic fatty acid and the anionic fatty acid are mixed together at an electric charge ratio of 1:1 in a composition consisting only of the cationic fatty acid and the anionic fatty acid.
(31) A composition for use in formation of a complex with a pharmaceutical ingredient, the composition containing a cation and an anion, wherein the cation is a cationic surfactant, and the anion is an anionic surfactant.
(32) The composition according to (31), wherein the cation is a cationic lipid, and the anion is a fatty acid.
(33) The composition according to (32), wherein the anion is a long-chain fatty acid (preferably, having 14 to 22 carbon atoms).
(34) The composition according to (33), wherein the anion is a long-chain unsaturated fatty acid (preferably having 14 to 22 carbon atoms).
(35) The composition according to (34), wherein the anion is linoleic acid or oleic acid.
(36) The composition according to any one of (31) to (35), wherein the cation is EDMPC.
(41) A composition containing:
   a population of solid complexes each containing a cation, an anion, and a nucleic acid; and
   an oil phase, wherein
   the population of the solid complexes is dispersed in the oil phase, and
   the cation is a cationic lipid and/or the anion is a fatty acid.
(42) The composition according to (41), wherein the anion is a long-chain fatty acid (preferably, having 14 to 22 carbon atoms).
(43) The composition according to (42), wherein the anion is a long-chain unsaturated fatty acid (preferably having 14 to 22 carbon atoms).
(44) The composition according to (43), wherein the anion is linoleic acid or oleic acid.
(45) The composition according to any one of (41) to (44), wherein the cation is EDMPC.
(46) The composition according to (41), wherein the population of the solid complexes includes a solid complex of about 5 nm to about 50 nm.
(47) The composition according to (42), wherein the population of the solid complexes includes a solid complex of about 5 nm to about 50 nm.
(48) The composition according to (43), wherein the population of the solid complexes includes a solid complex of about 5 nm to about 50 nm.
(49) The composition according to (44), wherein the population of the solid complexes includes a solid complex of about 5 nm to about 50 nm.
(50) The composition according to (45), wherein the population of the solid complexes includes a solid complex of about 5 nm to about 50 nm.
(51) The composition according to (46), wherein the population of the solid complexes includes a structure including a plurality of aggregates (e.g., diameter: 100 nm to 500 nm) of solid complexes of about 5 nm to about 50 nm.
(52) The composition according to (47), wherein the population of the solid complexes includes a structure including a plurality of aggregates (e.g., diameter: 100 nm to 500 nm) of solid complexes of about 5 nm to about 50 nm.
(53) The composition according to (48), wherein the population of the solid complexes includes a structure including a plurality of aggregates (e.g., diameter: 100 nm to 500 nm) of solid complexes of about 5 nm to about 50 nm.
(54) The composition according to (49), wherein the population of the solid complexes includes a structure including a plurality of aggregates (e.g., diameter: 100 nm to 500 nm) of solid complexes of about 5 nm to about 50 nm.
(55) The composition according to (50), wherein the population of the solid complexes includes a structure including a plurality of aggregates (e.g., diameter: 100 nm to 500 nm) of solid complexes of about 5 nm to about 50 nm.
(56) The composition according to any one of the above embodiments, wherein the cation and the anion are contained at an electric charge ratio of 1:0.8 to 1:1.25.
(57) The composition according to any one of the above embodiments, wherein the cation and the anion are capable of forming an ionic liquid at least when the cation and the anion are mixed together at an electric charge ratio of 1:1 in a composition consisting only of the cation and the anion.
(61) A composition for use in formation of a complex with a pharmaceutical ingredient, the composition containing a mixture containing a cation and an anion, wherein the mixture is capable of forming an ionic liquid at least in a composition containing only the cation and the anion at an electric charge ratio of 1:1 or the composition itself is an ionic liquid, and the composition consists of the cation and the anion or consists of the cation, the anion, and a solvent.
(62) The composition according to any one of (31) to (36), for use in enhancing skin permeation, the composition containing a mixture containing a cation and an anion, wherein the mixture is capable of forming an ionic liquid at least in a composition containing only the cation and the anion at an electric charge ratio of 1:1 or the composition itself is an ionic liquid, and the composition consists of the cation and the anion or consists of the cation, the anion, and a solvent.
(63) The composition according to (61) or (62), wherein the solvent contains a volatile organic solvent or is a volatile organic solvent.
(64) The composition according to any one of the above embodiments for transdermal administration, for use in enhancing skin permeation, the composition containing a mixture containing a cation and an anion, and a solvent, wherein the solvent contains a volatile organic solvent or is a volatile organic solvent.
(65) The composition according to (64), wherein the volatile organic solvent is a lower alcohol or contains a lower alcohol.
(66) The composition according to (65), wherein the lower alcohol is an alcohol having one to four carbon atoms.
(67) The composition according to (66), wherein the lower alcohol is ethanol.
(68) The composition according to any one of (61) to (67), wherein the cation is a cationic lipid (preferably, a cationic long-chain lipid).
(69) The composition according to any one of (61) to (68), wherein the anion is a fatty acid.
(70) The composition according to (68), wherein the cationic lipid is EDMPC.
(71) The composition according to any one of (61) to (670), wherein the anion is a long-chain fatty acid (e.g., having 14 to 22 carbon atoms).
(72) The composition according to (71), wherein the anion is a long-chain unsaturated fatty acid **(e.g.,** having 14 to 22 carbon atoms).
(73) The composition according to (72), wherein the anion is linoleic acid or oleic acid.
(81) A composition containing: solid complexes containing a surfactant **(e.g.,** a nonionic surfactant **(e.g.,** ER-290), a cationic surfactant, an anionic surfactant) and a pharmaceutical agent; and an oil phase, wherein the oil phase contains an anionic long-chain lipid, and the solid complexes are dispersed in the oil phase.
(82) The composition according to (81), wherein the anionic long-chain lipid is a long-chain fatty acid.
(83) The composition according to (81) or (82), wherein the anionic long-chain lipid is a long-chain unsaturated fatty acid.
(84) The composition according to any one of (81) to (83), wherein the anionic long-chain lipid is linoleic acid.
(85) The composition according to any one of (81) to (84) for transdermal administration (i.e., for use in transdermal administration).
(91) The composition according to any one of the above embodiments, wherein the cation is a surfactant having an HLB value of 10 or less, preferably of about 1 to 4**.**
(92) The composition according to any one of the above embodiments, wherein the anion is a surfactant having an HLB value of 10 or less, preferably of about 1 to 4.
(93) The composition according to any one of the above embodiments, wherein the cation and the anion are each a surfactant having an HLB value of 10 or less, preferably of about 1 to 4.
(101) The composition according to any one of the above embodiments, wherein the oil phase further contains an anionic surfactant.
(102) The composition according to any one of the above embodiments, wherein the oil phase further contains an anionic surfactant, whereby the skin permeation of a drug such as a nucleic acid is enhanced as compared with the composition with the oil phase containing no additional anionic surfactant.
(103) The composition according to (101) or (102), wherein the anionic surfactant further contains a fatty acid (preferably, a long-chain fatty acid, more preferably, a long-chain unsaturated fatty acid).

### Brief Description of Drawings

[Figure 1] Figure 1 is a series of ¹H-NMR spectra of compositions (each formed an ionic liquid) each consisting of a cationic surfactant and an anionic surfactant that are synthesized.
[Figure 2] Figure 2 shows a process in which cation-anion-nucleic acid complexes are formed through an emulsion-forming step, freeze-dried, and then dispersed in isopropyl myristate (IPM) to give a dispersion with solid complexes dispersed in oil.
[Figure 3A] Figure 3A shows the appearances of dispersions obtained by dispersing solid complexes of a mixture of a cationic surfactant and an anionic surfactant; and a nucleic acid.
[Figure 3B] Figure 3B shows the particle size distributions of solid complexes in the obtained dispersions in dynamic light scattering (DLS) measurement.
[Figure 3C] Figure 3C shows the time-dependent changes of particle sizes and polydispersity indexes (PDI) of the solid complexes in the obtained dispersions.
[Figure 4] Figure 4 shows the influence of nucleic acid and surfactant concentrations on solid complexes obtained.
[Figure 5] Figure 5 shows results of evaluation of the cutaneous permeation of the obtained complexes.
[Figure 6] Figure 6 shows the solubilities of lipids or lipid mixtures in cyclohexane and the appearances of dispersions.
[Figure 7] Figure 7 shows results of evaluation of the permeation of the obtained complexes into hairless mouse skin.
[Figure 8] Figure 8 shows the influence of addition of ionic surfactants to the skin permeation of a dispersion containing ER-290-nucleic acid solid complexes.
[Figure 9] Figure 9 shows the influences of ionic surfactants on horny layer lipid.
[Figure 10] Figure 10 shows results of molecular simulation for horny layer lipid to which ionic surfactants were applied.
[Figure 11] Figure 11 shows a scheme of a method for preparing surfactant-pharmaceutical agent complexes by means of an ethanol dilution method.
[Figure 12] Figure 12 shows the influence of the ionicities of surfactants on the particle size distribution of solid complexes obtained by the ethanol dilution method.
[Figure 13] Figure 13 shows the particle size distributions of solid complexes formed with an emulsion-forming method (Pre-method) and those formed by the ethanol dilution method (New-method).
[Figure 14] Figure 14 shows the cutaneous permeation and penetration of nucleic acid-containing solid complexes formed with the emulsion-forming method (Pre-method) and those formed by the ethanol dilution method (New-method).
[Figure 15A] Figure 15A shows the stability of the appearance of a dispersion of solid complexes formed by the ethanol dilution method.
[Figure 15B] Figure 15B shows the stability of the particle size distribution of a dispersion of solid complexes formed with the ethanol dilution method.
[Figure 16] Figure 16 shows the influence of mixing ratios between a nucleic acid and ionic lipids on the particle size distribution of solid complexes formed by the ethanol dilution method.
[Figure 17] Figure 17 shows the influence of mixing ratios between a nucleic acid and ionic lipids on the cutaneous permeation and penetration of solid complexes formed with the ethanol dilution method.
[Figure 18] Figure 18 shows the influence of mixing ratios between a nucleic acid and ionic lipids on the particle size distribution of solid complexes formed with the ethanol dilution method.
[Figure 19] Figure 19 shows the influence of mixing ratios between a nucleic acid and ionic lipids on the zeta potential of solid complexes formed with the ethanol dilution method.
[Figure 20] Figure 20 shows the relationship between stirring time and particle formation after mixing a drug solution and ionic lipids with the ethanol dilution method.
[Figure 21] Figure 21 shows the relationship between temperature conditions in a step of mixing a drug solution and ionic lipids by the ethanol dilution method and particle formation.
[Figure 22] Figure 22 shows the relationship between ratios of an aqueous solution and an ethanol solution mixed by the ethanol dilution method and particle size distribution.
[Figure 23] Figure 23 shows the influence of lipids for use on the particle size distribution of solid complexes formed by the ethanol dilution method.
[Figure 24] Figure 24 shows the particle size distribution and zeta potential of solid complexes formed by the ethanol dilution method in the case where the cation is (CH₃)₃N⁺(CH₂)₂OH (choline) and the anion is oleic acid.
[Figure 25] Figure 25 shows the cutaneous permeation and penetration of different solid complexes formed by the ethanol dilution method.
[Figure 26] Figure 26 shows the particle size distribution and stability of peptide nucleic acid-containing solid complexes formed by the ethanol dilution method.
[Figure 27] Figure 27 shows the particle size distribution and stability of protein-containing solid complexes formed by the ethanol dilution method.
[Figure 28] Figure 28 shows the cutaneous permeation of solid complexes, containing ionic surfactants and a protein, formed by the ethanol dilution method.
[Figure 29] Figure 29 shows transmission electron microscopy images of an ionic lipid-nucleic acid solid complex formed by an emulsion method (cyclohexane method) and those formed by the ethanol dilution method.
[Figure 30] Figure 30 shows results of transfection experiment with ionic surfactant-nucleic acid complexes formed by the ethanol dilution method for cultured cells.
[Figure 31] Figure 31 shows fluorescence microscopy images of a skin section that demonstrate the permeation of ionic surfactant-nucleic acid complexes formed by the ethanol dilution method.
[Figure 32] Figure 32 shows expression of introduced mRNA in cultured cells after transfection of the cultured cells with an aqueous solution containing ionic surfactant-mRNA complexes formed by the ethanol dilution method.
[Figure 33] Figure 33 shows results of evaluation of the cytotoxicity of different ionic lipid-nucleic acid solid complexes formed by the ethanol dilution method.
[Figure 34] Figure 34 shows the knock-down effect of hybrid particles containing ASO in different tissues.

### Description of Embodiments

Embodiments according to the present invention will be described with reference to the drawings. The present invention is not limited to the embodiments shown below and the drawings. The meaning of the expression "have", "include (comprise)"**,** or "contain (comprise)" in the embodiments shown below includes the meaning of "consist of" or "be composed of".

The term "cation" herein means a molecule having a positive charge under a condition of pH 7. The term "cationic" herein means a characteristic of having a positive charge under a condition of pH 7. The term "anion" herein means a molecule having a negative charge under a condition of pH 7. The term "anionic" herein means a characteristic of having a negative charge under a condition of pH 7.

A "surfactant" herein is a molecule having a relatively hydrophilic moiety and a relatively hydrophobic moiety in the molecule. Surfactants are roughly classified into ionic surfactants and nonionic surfactants. Ionic surfactants include cationic surfactants and anionic surfactants. Typically, an ionic surfactant can have either one of a cation and an anion and a hydrophobic moiety. Each cationic surfactant may have a cation moiety and an aliphatic chain (e.g., a long-chain aliphatic chain) such as alkyl, alkenyl, or alkynyl. Each anionic surfactant may have an anion moiety and a fatty acid such as a long-chain fatty acid, an unsaturated fatty acid (e.g., monovalent or divalent), or a long-chain unsaturated fatty acid (e.g., monovalent or divalent). "Long-chain" is a word given to a compound having 14 to 22 carbon atoms. An aliphatic chain can be an aliphatic chain having 8 to 22 carbon atoms, that is, a C₈ to C₂₂ aliphatic chain such as C₈ to C₂₂ alkyl, C₈ to C₂₂ alkenyl, or C₈ to C₂₂ alkynyl. An aliphatic chain can be a long-chain aliphatic chain. The term saturated means that the aliphatic chain has neither a double bond nor a triple bond, and the term unsaturated means that the aliphatic chain has at least one double bond or triple bond. While aliphatic chains having a double bond include cis-forms and trans-forms, cis-forms are considered preferable for biocompatibility.

Lipids are biogenic molecules soluble in nonpolar solvents. Lipids can be classified into glycerolipids, glycerophospholipids, sphingolipids, sterol lipids, pronol lipids, glycolipids, and polyketides. Glycerolipid is a collective term for molecules in each of which a fatty acid is ester-bonded to a hydroxy group of glycerol. Glycerol has three hydroxy groups, and up to three fatty acids can be linked thereto through ester bonds. In glycerophospholipids, two fatty acids are ester-bonded to two hydroxy groups of glycerol, and a phosphate group is linked to one hydroxy group. A sphingolipid can be ceramide, sphingophospholipid, or sphingoglycolipid. Sterol lipids are a subgroup of terpenoid. A sterol lipid can be sterol (e.g., cholesterol). Prenol lipids are terpenoids other than sterol lipids.

Cationic lipids are descried in U.S. Patent Application Publication Nos. 2006/0083780 and 2006/0240554, U.S. Patent Nos. 5,208,036, 5,264,618, 5,279,833, 5,283,185, 5,753,613, and 5,785,992, and PCT Publication No. WO 96/10390, and the disclosures of them are totally incorporated herein by reference for all purposes. The cationic lipid is, for example, 1,2-dilinoleyloxy-N,N-dimethylaminopropane (DLinDMA), 1,2-dilinolenyloxy-N,N-dimethylaminopropane (DLenDMA), 2,2-dilinoleyl-4-(2-dimethylaminoethyl)-[1,3]-dioxolane (DLin-K-C2-DMA; "XTC2"), 2,2-dilinoleyl-4-(3-dimethylaminopropyl)-[1,3]-dioxolane (DLin-K-C3-DMA), 2,2-dilinoleyl-4-(4-dimethylaminobutyl)-[1,3]-dioxolane (DLin-K-C4-DMA), 2,2-dilinoleyl-5-dimethylaminomethyl-[1,3]-dioxane (DLin-K6-DMA), 2,2-dilinoleyl-4-N-methylpepiazino-[1,3]-dioxolane (DLin-K-MPZ), 2,2-dilinoleyl-4-dimethylaminomethyl-[1,3]-dioxolane (DLin-K-DMA), 1,2-dilinoleylcarbamoyloxy-3-dimethylaminopropane (DLin-C-DAP), 1,2-dilinoleyloxy-3-(dimethylamino)acetoxypropane (DLin-DAC), 1,2-dilinoleyloxy-3-morpholinopropane (DLin-MA), 1,2-dilinoleoyl-3-dimethylaminopropane (DLinDAP), 1,2-dilinoleylthio-3-dimethylaminopropane (DLin-S- DMA), 1-linoleoyl-2-linoleyloxy-3-dimethylaminopropane (DLin-2-DMAP), 1,2-dilinoleyloxy-3-trimethylaminopropane chloride salt (DLin-TMA.Cl), 1,2-dilinoleoyl-3-trimethylaminopropane chloride salt (DLin-TAP.Cl), 1,2-dilinoleyloxy-3-(N-methylpiperazino)propane (DLin-MPZ), 3-(N,N-dilinoleylamino)-1,2-propanediol (DLinAP), 3-(N,N-dioleylamino)-1,2-propanediol (DOAP), 1,2-dilinoleyloxo-3-(2-N,N-dimethylamino)ethoxypropane (DLin-EG-DMA), N,N-dioleyl-N,N-dimethylammonium chloride (DODAC), 1,2-dioleyloxy-N,N-dimethylaminopropane (DODMA), 1,2-distearyloxy-N,N-dimethylaminopropane (DSDMA), N-(1-(2,3-dioleyloxy)propyl)-N,N,N-trimethylammonium chloride (DOTMA), N,N-distearyl-N,N-dimethylammonium bromide (DDAB), N-(1-(2,3-dioleoyloxy)propyl)-N,N,N-trimethylammonium chloride (DOTAP), 3-(N-(N',N'-dimethylaminoethane)-carbamoyl)cholesterol (DC-Chol), N-(1,2-dimyristyloxyprop-3-yl)-N,N-dimethyl-N-hydroxyethylammonium bromide (DMRIE), 2,3-dioleyloxy-N-[2(spermine-carboxamide)ethyl]-N,N-dimethyl-1-propanaminium trifluoroacetate (DOSPA), dioctadecylamidoglycylspermine (DOGS), 3-dimethylamino-2-(cholest-5-en-3-β-oxybutan-4-oxy)-1-(cis,cis-9,12-octadecadienoxy)propane (CLinDMA), 2-[5'-(cholest-5-en-3-β-oxy)-3'-oxapentoxy)-3-dimethyl-1-(cis,cis-9',1-2'-octadecadienoxy)propane (CpLinDMA), N,N-dimethyl-3,4-dioleyloxybenzylamine (DMOBA), 1,2-N,N'-dioleylcarbamyl-3-dimethylaminopropane (DOcarbDAP), 1,2-N,N'-dilinoleylcarbamyl-3-dimethylaminopropane (DLincarbDAP), or a mixture of any of them. In a preferred embodiment, the positive ionic lipid is DLinDMA, DLin-K-C2-DMA ("XTC2"), or a mixture of them. For example, the cationic lipid is preferably 1,2-dimyristoyl-sn-glycero-3-ethyl-phosphatidylcholine (EDMPC).

A preferred example of the anionic lipid is an anionic lipid represented by general formula (1):

R-COO-X⁺ ... (1).

In general formula (1), R represents a substituted or unsubstituted alkyl group or a substituted or unsubstituted alkenyl group, and at least one ethylene group constituting the alkenyl group may be replaced with a vinylene group.

The alkyl group for R may be linear, branched, or cyclic. The number of carbon atoms of the alkyl group for R is preferably 8 to 22, more preferably 12 to 22, and even more preferably 14 to 22. Examples of the alkyl group for R can include linear alkyl groups such as a dodecyl group, a tridecyl group, a tetradecyl group, a pentadecyl group, a hexadecyl group, a heptadecyl group, an octadecyl group, a nonadecyl group, an icosyl group, a henicosyl group, and a docosyl group, branched alkyl groups thereof, and cyclic alkyl groups thereof. Substituents that can substitute the alkyl group can include an amino group, a benzyl group, and halogen atoms (e.g., a fluorine atom). Those substituents may be further substituted with a substituent. In the case where the alkyl group is substituted with a substituent, the sum total of the number of carbon atoms of the alkyl group and the number of carbon atoms of the substituent is preferably 8 to 22, more preferably 12 to 22, and even more preferably 14 to 22.

The alkenyl group for R may be linear, or branched. The number of carbon atoms of the alkenyl group for R is preferably 8 to 22, more preferably 12 to 22, and even more preferably 14 to 22. Examples of the alkenyl group for R can include linear alkenyl groups such as a dodecenyl group, a tridecenyl group, a tetradecenyl group, a pentadecenyl group, a hexadecenyl group, a heptadecenyl group, an octadecenyl group, a nonadecenyl group, an icosenyl group, a henicosenyl group, and a docosenyl group, branched alkenyl groups thereof. Substituents that can substitute the alkenyl group can include an amino group, a benzyl group, and halogen atoms (e.g., a fluorine atom). Those substituents may be further substituted with a substituent. In the case where the alkenyl group is substituted with a substituent, the sum total of the number of carbon atoms of the alkenyl group and the number of carbon atoms of the substituent is preferably 8 to 22, more preferably 12 to 22, and even more preferably 14 to 22.

In the alkenyl group for R, at least one ethylene group may be replaced with a vinylene group to form a polyene structure. The number of double bonds in the polyene structure is preferably 2 to 6, and more preferably 2 to 4. The positions of double bonds are not limited, but it is preferable that double bonds be positioned with at least two single bonds intervening between two double bonds. The double bond are preferably in the cis-form. Preferably, one or more, more preferably two or more, even more preferably three or more double bonds can be contained in R. Among these, R is preferably a group having an unsaturated bond, and more preferably a substituted or unsubstituted alkenyl group or a group having a substituted or unsubstituted polyene structure.

The alkyl group, the alkenyl group, and the group having a polyene structure for R may be substituted with a substituent, and even in this case it is preferable for R to consist only of carbon atoms and hydrogen atoms. That is, in the case where the alkyl group, the alkenyl group, and the polyene structure are substituted with a substituent, it is preferable also for the substituent to consist only of carbon atoms and hydrogen atoms.

For R-COO⁻, for example, a carboxylate ion, which is generated as a result of the dissociation of a proton from the carboxy group of a fatty acid, or a derivative thereof can be used. The fatty acid to generate a carboxylate ion may be a saturated fatty acid or an unsaturated fatty acid, and may be preferably an unsaturated fatty acid, more preferably a cis unsaturated fatty acid, and even more preferably a cis long-chain unsaturated fatty acid. Examples of the saturated fatty acid can include myristic acid (C14:0), palmitic acid (C16:0), stearic acid (C18:0), and lauric acid (C12:0); examples of the unsaturated fatty acid can include oleic acid (C18:1), linoleic acid (C18:2), α-linolenic acid (C18:3), γ-linolenic acid (C18:3), arachidonic acid (C20:4), icosapentaenoic acid (C20:5), docosahexaenoic acid (C22:6), and erucic acid (C22:1). Here, numerical values in parentheses indicate the number of carbon atoms and the number of double bonds in the corresponding fatty acid. For example, (C18:2) for linoleic acid indicates that the number of carbon atoms is 18 and the number of double bonds is 2. In general formula (1), X⁺ represents a phospholipid having a cationic group. Here, the term "phospholipid" means a lipid containing a phosphate structure, and the term "cationic group" means a substituent with positive electricity. X⁺ is preferably a glycerophospholipid having a cationic group, and more preferably a derivative of phosphatidylcholine. Here, the derivative of phosphatidylcholine is a compound having a glycerol skeleton, substituted or unsubstituted alkanoyl groups bonding to positions 1 and 2 of the glycerol skeleton, a phosphate group (-P(=O)(OH)O⁻) bonding to position 3 of the glycerol skeleton, and a choline residue bonding to the phosphate group. In this phosphatidylcholine derivative, at least one ethylene group of the alkanoyl groups may be replaced with a vinylene group, and the hydrogen atom of the hydroxy group of the phosphate group may be replaced with a substituted or unsubstituted alkyl group.

X⁺ is preferably a phospholipid having a structure represented by general formula (2), and more preferably a glycerophospholipid having a structure represented by general formula (2):

CH(-CH₂₋O-C(=O)-R¹⁰)(-O-C(=O)-R¹¹)-CH₂₋O-P(OR¹)(OR²)(=O)··· (2).

In general formula (2), R¹ represents an alkyl group substituted with a cationic group, and R² represents a hydrogen atom or a substituted or unsubstituted alkyl group. The phospholipid having the structure represented by general formula (2) has a positive charge as a whole, and preferably lacks a negatively charged moiety.

The alkyl group for R¹ may be linear, branched, or cyclic. The number of carbon atoms of the alkyl group for R¹ is preferably 1 to 20, more preferably 1 to 10, and even more preferably 1 to 6. Examples of the alkyl group for R¹ can include a methyl group, an ethyl group, an n-propyl group, and an isopropyl group, and the alkyl group for R¹ is preferably an ethyl group.

The cationic group in R¹ is preferably an ammonium group represented by general formula (3):

-NR⁴R⁵R⁶⁺ ··· (3).

In general formula (3), R⁴, R⁵, and R⁶ each independently represent a hydrogen atom or a substituted or unsubstituted alkyl group, and * indicates the position of bonding to the alkyl group. R⁴, R⁵, and R⁶ may be identical or different. The number of substituted or unsubstituted alkyl groups among R⁴, R⁵, and R⁶ is not limited, and all of R⁴, R⁵, and R⁶ may be hydrogen atoms or substituted or unsubstituted alkyl groups; one or two of R⁴, R⁵, and R⁶ may be a hydrogen atom(s) with the rest being a substituted or unsubstituted alkyl group(s).

The alkyl group for R⁴, R⁵, and R⁶ may be linear, branched, or cyclic. The number of carbon atoms of the alkyl group for R⁴, R⁵, and R⁶ is preferably 1 to 20, more preferably 1 to 10, and even more preferably 1 to 6. Examples of the alkyl group for R⁴, R⁵, and R⁶ can include a methyl group, an ethyl group, an n-propyl group, and an isopropyl group, and the alkyl group for R⁴, R⁵, and R⁶ is preferably a methyl group. Examples of substituent that can substitute the alkyl group can include a benzyl group and halogen atoms **(e.g.,** a fluorine atom). Those substituents may be further substituted with a substituent. In a preferred embodiment, R⁴, R⁵, and R⁶ are each a methyl group.

The cationic group in R¹ is preferably a quaternary ammonium group, and more preferably a quaternary ammonium group represented by general formula (5) (an ammonium group such that all R⁴ groups are substituted or unsubstituted alkyl groups). The position of substitution with the cationic group in the alkyl group is not limited, and it is preferable that a hydrogen atom bonding to a terminal carbon atom of the alkyl group be replaced with the cationic group.

In general formula (2), R² represents a hydrogen atom or a substituted or unsubstituted alkyl group. It is preferable that R² be a substituted or unsubstituted alkyl group. The alkyl group for R² may be linear, branched, or cyclic. The number of carbon atoms of the alkyl group for R² is preferably 1 to 20, more preferably 1 to 10, and even more preferably 1 to 6. Examples of the alkyl group for R² can include a methyl group, an ethyl group, an n-propyl group, and an isopropyl group, and the alkyl group for R² is preferably an ethyl group. Substituents that can substitute the alkyl group can include a benzyl group and halogen atoms (e.g., a fluorine atom). Those substituents may be further substituted with a substituent.

In general formula (2), R¹⁰ and R¹¹ each independently represent an aliphatic chain having 8 to 22 carbon atoms, are preferably each a long-chain aliphatic chain, and can be each a long-chain saturated aliphatic chain or a long-chain unsaturated aliphatic chain. In an embodiment, R¹⁰ and R¹¹ are each independently a long-chain saturated aliphatic chain having 12 to 22 carbon atoms. In an embodiment, R¹⁰ and R¹¹ can be each a long-chain saturated aliphatic chain having 12 to 14 (e.g., 13) carbon atoms. In an embodiment, the glycerophospholipid represented by general formula (2) can be EDMPC.

The number of substitutions with an alkylcarbonyloxy group in the alkyl group is preferably 2 to 8, more preferably 2 to 4, and most preferably 2. The positions of substitutions with an alkylcarbonyloxy group in the alkyl group are not limited; in the case where the alkyl group as the main chain of R³ is an n-propyl group, a product such that a hydrogen atom bonding to the terminal carbon atom and a hydrogen atom bonding to the adjacent carbon atom are each replaced with an alkylcarbonyloxy group corresponds to a glycerophospholipid, which is a particularly preferred phospholipid.

For description, a preferred scope, and specific examples of the alkyl group in each alkylcarbonyloxy group, reference can be made to the description, preferred scope, and specific examples of the alkyl group for R in the above.

The following shows preferred examples of the compound having the structure represented by general formula (1); however, the ionic surfactants according to the present embodiment should not be interpreted in a limited manner with reference to those specific examples. For example, the carboxylate ion is a long-chain fatty acid, preferably a long-chain unsaturated fatty acid, for example, linoleic acid or oleic acid, or a long-chain saturated fatty acid, and can be, for example, stearic acid; in the glycerophospholipid represented by general formula (2), R¹ is -NR⁴R⁵R⁶, R⁴, R⁵, and R⁶ are each independently methyl or ethyl, preferably each methyl, and R¹⁰ and R¹¹ are each independently a long-chain aliphatic chain, preferably each independently a long-chain aliphatic chain having 12 to 22 carbon atoms, more preferably a long-chain aliphatic chain having 12 to 14 carbon atoms. In a more specific example, the carboxylate ion is a long-chain unsaturated fatty acid, for example, linoleic acid or oleic acid; in the glycerophospholipid represented by general formula (2), R¹ is -NR⁴R⁵R⁶, R⁴, R⁵, and R⁶ are each independently methyl or ethyl, preferably each methyl, and R¹⁰ and R¹¹ are each independently a long-chain aliphatic chain having 12 to 22 carbon atoms, more preferably a long-chain aliphatic chain having 12 to 14 carbon atoms. In a much more specific example, the carboxylate ion is a long-chain unsaturated fatty acid, for example, linoleic acid or oleic acid; in the glycerophospholipid represented by general formula (2), R¹ is -NR⁴R⁵R⁶, R⁴, R⁵, and R⁶ are each methyl, and R¹⁰ and R¹¹ are each independently a long-chain aliphatic chain having 12 to 22 carbon atoms, more preferably a long-chain aliphatic chain having 12 to 14 carbon atoms. A much more specific example is a formula given below, wherein R-COO⁻ is synonymous with R-COO⁻ in general formula (1), and specific examples thereof include a carboxylate ion of linoleic acid, oleic acid, acetic acid, or stearic acid.

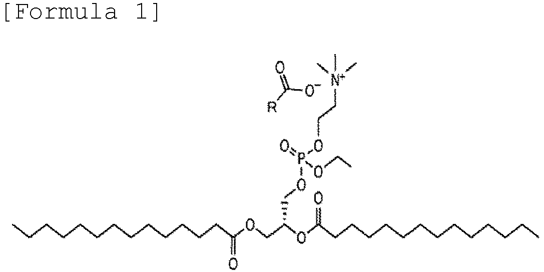

The formula represents a situation that a carboxylate ion and a preferred cationic surfactant are forming an ionic bond. The mixture of a cationic lipid and an anionic lipid can be an ionic liquid at normal temperature when mixed at an electric charge ratio of 1:1.

Examples of the anionic surfactant include a phospholipid and a fatty acid. Examples of the anionic surfactant include phosphatidylglycerol, cardiolipin, diacylphosphatidylserine, diacylphosphatidic acid, N-dodecanoylphosphatidylethanolamine, N-succinylphosphatidylethanolamine, N-glutarylphosphatidylethanolamine, lysylphosphatidylglycerol, palmitoyloleoylphosphatidylglycerol (POPG), and other compounds with a negative-ionic modifying group and a neutral lipid bonding together.

Examples of the phospholipid include phosphatidylcholine, phosphatidylethanolamine, phosphatidylserine**,** phosphatidylinositol, phosphatidic acid, palmitoyloleoylphosphatidylcholine, lysophosphatidylcholine, lysophosphatidylethanolamine, dipalmitoylphosphatidylcholine, dioleoylphosphatidylcholine, distearoylphosphatidylcholine, and dilinoleoylphosphatidylcholine.

The fatty acid is a carbon chain having a monovalent carboxylic acid. The fatty acid is preferably not in the trans-form but in the cis-form. The fatty acid is represented by the rational formula CH₃-R-CO₂H {wherein, R is a carbon chain}, and specified on the basis of the numbers of carbon atoms and double bonds contained in the fatty acid. For example, capric acid has R of -(CH₂)₈- and is specified with 10:0, lauric acid has R of -(CH₂)₁₀- and is specified with 12:0, myristic acid has R of - (CH₂)₁₄- and is specified with 14:0, pentadecylic acid has R of -(CH₂)₁₅- and is specified with 15:0, palmitic acid has R of -(CH₂)₁₆- and is specified with 16:0, palmitoleic acid has R of -(CH₂)₅CH=CH(CH₂)₇- and is specified with 16:1, stearic acid has R of -(CH₂)₁₈- and is specified with 18:0, oleic acid has R of -(CH₂)₇CH=CH(CH₂)₇- and is specified with 18:1(n-9), vaccenic acid has R of - (CH₂)₅CH=CH(CH₂)₉- and is specified with 18:1(n-7), linoleic acid has R of -(CH₂)₃(CH₂CH=CH)₂(CH₂)₇- and is specified with 18:2(n-6), (9,12,15)-linolenic acid has R of -(CH₂CH=CH)₃(CH₂)₇- and is specified with 18:3(n-3), and (6,9,12)-linolenic acid has R of - (CH₂)₃(CH₂CH=CH)₃(CH₂)₄- and is specified with 18:3(n-6).

A "solid complex" is herein a complex consisting of a solid content. For example, freeze-dried complexes can be maintained in a dispersed state in an oil layer. Each solid complex may contain water, but is a complex containing a solid component. Solid complex may have a low moisture content, and the moisture content as measured with a known method can be, for example, 1% by mass or less, 0.9% by mass or less, 0.8% by mass or less, 0.7% by mass or less, 0.6% by mass or less, 0.5% by mass or less, 0.4% by mass or less, 0.3% by mass or less, 0.2% by mass or less, or 0.1% by mass or less.

### (Embodiments)

The present invention provides a percutaneous absorption formulation that allows molecule of interest (e.g., a nucleic acid) to be transdermally delivered into a living body. The percutaneous absorption formulation of the present invention can achieve an amount of cutaneous permeation at least about 1.5 times or more, about 2 times or more, about 2.5 times or more, about 3 times or more, about 3.5 times or more, or about 4 times or more that of molecule of interest (e.g., a nucleic acid) dissolved in a buffer solution (e.g., Tris EDTA (TE) buffer solution). The TE buffer solution typically contains 10 mM Tris (pH 7.4 to 8.0) and 1 mM EDTA. The percutaneous absorption formulation of the present invention can achieve an amount of cutaneous permeation at least about 5 times or more, about 6 times or more, about 7 times or more, about 8 times or more, about 9 times or more, about 10 times or more, about 11 times or more, about 12 times or more, about 13 times or more, about 14 times or more, or about 15 times or more, compared to that of molecule of interest (e.g., a nucleic acid) dissolved in a buffer solution (e.g., Tris EDTA buffer solution (TE buffer solution)). In an embodiment, the S/O formulation of the present invention achieves an increased amount of cutaneous permeation of molecule of interest (e.g., a nucleic acid) in a manner depending on any one or more of the structure of particles, the charged state, and added components. Molecule of interest is herein molecules that can give benefits when delivered with the method of the present invention.

The present invention provides a composition containing: solid complexes each containing a molecule of interest (e.g., a nucleic acid) and a surfactant; and an oil phase. In a preferred embodiment, the composition contains no aqueous phase. In the composition, the complexes are dispersed in the oil phase. An aqueous phase containing molecule of interest (e.g., a nucleic acid) and an organic solvent phase containing a surfactant are mixed together to form a W/O emulsion, and the resulting W/O emulsion is freeze-dried to remove the moisture and organic solvent component, giving solid complexes in a dry state. In the W/O emulsion, particles in which an aqueous phase containing a molecule of interest (e.g., a nucleic acid) is covered with a surfactant are believed to be dispersed in the organic phase. Accordingly, in the solid complexes obtained by drying the particles, a molecule of interest (e.g., a nucleic acid) is expected to be covered with a surfactant. Thus, in an embodiment, each solid complex contains a molecule of interest (e.g., a nucleic acid) and a surfactant, and the molecule of interest (e.g., a nucleic acid) is covered with the surfactant. In normal cases, covering is achieved with a sufficient amount (in particular, a saturating amount) of the surfactant over the entire surface of the core containing a molecule of interest (e.g., a nucleic acid). In the composition or formulation of the present invention, the solid complexes are dispersed in the oil phase. The surfactant is believed to bond to a molecule of interest (e.g., a nucleic acid) at the hydrophilic moiety and be in contact with the oil phase with the hydrophobic moiety facing the outside of the complex. In this way, the surfactant probably plays an important role in promoting the solid complexes containing a molecule of interest (e.g., a nucleic acid), which is hydrophilic, to be dispersed in the oil phase. The composition is obtained by dispersing the resulting solid complexes in an oil phase. The complexes are expected to be forming solid particles or solid complexes in the composition. The composition of the present invention can be used for allowing molecule of interest (e.g., a nucleic acid) to be transdermally absorbed. Accordingly, the composition of the present invention can be formulated into a percutaneous absorption agent. Typically, in the complexes of the present invention, the surfactant can account for about 50 w/w% or more, about 60 w/w% or more, about 70 w/w% or more, about 80 w/w% or more, about 90 w/w% or more, about 95 w/w% or more, or about 100 w/w% of components other than a molecule of interest (e.g., a nucleic acid). In a preferred embodiment, each of the complexes consists of a molecule of interest (e.g., a nucleic acid) and a surfactant {however, the complexes can be contaminated with an inevitable impurity from the production process}. The composition can be a dermatologically acceptable composition.

In an embodiment, a composition is provided, the composition containing: complexes (preferably complex particles or solid complexes) containing a molecule of interest (e.g., a nucleic acid) and a surfactant; and an aqueous phase. In a preferred embodiment, the composition contains no oil phase. In the composition, the complexes are dispersed in the aqueous phase. An aqueous phase containing molecule of interest (e.g., a nucleic acid) and an organic solvent phase (in particular, lower alcohol) containing a surfactant are mixed together to give a dispersion (preferably an aqueous dispersion) with surfactant-molecule of interest (e.g., a nucleic acid) complexes and an aqueous phase, wherein the surfactant- molecule of interest (e.g., a nucleic acid) complexes are dispersed in the aqueous phase. From examination on zeta potential and DLS, a molecule of interest (e.g., a nucleic acid) is expected to be covered with a surfactant in the complex. It is preferable for the aqueous dispersion to have no cytotoxicity. The aqueous composition can be used for introducing molecule of interest (e.g., a nucleic acid) into cultured cells.

The size of the complexes may be any size, without limitation, that allows the complex to keep the dispersed state in the oil phase and does not deteriorate the skin permeation of the complex. The average particle diameter (average particle diameter based on DLS) of the complex (complex particles) is, for example, about 1 nm to about 3000 nm, about 1 nm to about 2000 nm, about 1 nm to about 1500 nm, about 1 nm to about 1400 nm, about 1 nm to about 1300 nm, about 1 nm to about 1200 nm, about 1 nm to about 1100 nm, about 1 nm to about 1000 nm, preferably about 10 nm to about 900 nm, more preferably about 100 nm to about 700 nm, and even more preferably about 200 nm to about 500 nm {here, in the case where the nucleic acid is mRNA, the average particle diameter can be about 1 µm or more; in the case where the nucleic acid is antisense oligo or siRNA, the average particle size can be about 1 µm or less.}. In an embodiment, the average particle diameter of the complexes is about 300 nm to about 500 nm. The polydispersity index (PDI) is a measure of heterogeneity of the particle sizes of particles. Smaller PDI indicates that the particle sizes are homogenous. The polydispersity index of the formulation of the present invention is, for example, about 1 or less, and can be about 0.2 to about 0.8, about 0.3 to about 0.7, about 0.2 to 0.4, or about 0.4 to about 0.6.

Nucleic acids are hydrophilic compounds. Therefore, nucleic acids do not permeate into skin or have low cutaneous permeation when used alone in typical cases. In the composition or percutaneous absorption agent according to the present embodiment, a nucleic acid and a surfactant are allowed to form a complex to enhance the skin permeation of the nucleic acid. Likewise, hydrophilic compounds and hydrophilic proteins do not permeate into skin or have low cutaneous permeation when used alone in typical cases. In the composition or percutaneous absorption agent according to the present embodiment, a hydrophilic bioactive substance such as a hydrophilic compound and a hydrophilic protein, in place of a nucleic acid, forms a complex with a surfactant to enhance the skin permeation of the hydrophilic bioactive substance.

The nucleic acid may be DNA or RNA. The nucleic acid may be single-stranded or double-stranded. Examples of the nucleic acid include an antisense nucleic acid targeting mRNA or RNA (e.g., antisense oligo), shRNA, microRNA and siRNA, a decoy nucleic acid, which binds to a transcription factor to suppress the transcription stage, an aptamer which binds to a protein to inhibit the function, and CpG oligodeoxynucleotide, which acts on Toll-like receptor-9 to activate the immune system. The nucleic acid may be mRNA that expresses a specific protein in cells (mRNA vaccine). The nucleotide length of the nucleic acid is not limited, and, for example, about 10 to about 80 nucleotides, about 15 to about 50 nucleotides, or about 20 to about 30 nucleotides. The length of siRNA can be, for example, about 20 to about 30 nucleotides, about 20 to about 24 nucleotides, or about 21 to about 23 nucleotides. The nucleic acid may contain a modified nucleic acid in the nucleotide sequence. That is, the nucleic acid can be any of a nucleic acid such as DNA, RNA, and a hybrid of DNA and RNA, and a nucleic acid containing a modified nucleic acid (a gapmer and a mixmer). The gapmer is a single-stranded nucleic acid having a DNA moiety, and RNA or modified nucleic acid moieties, wherein the gapmer contains a single-stranded nucleic acid in which the RNA or modified nucleic acid moieties are present at both ends of the DNA moiety. The gapmer can be, for example, a double-stranded nucleic acid. The mixmer is a nucleic acid containing an antisense strand containing nucleic acids of different types. The sequence and length of the antisense oligo, microRNA, siRNA, shRNA, gapmer, mixer, or the like can be designed to exert a knock-down effect on target RNA.

Examples of the modified nucleic acid include a nucleic acid modified with a fluorescent dye, a biotinylated nucleic acid, and a nucleic acid with a cholesteryl group introduced therein. To enhance the stability of RNA, the nucleotides may be subjected to 2'-O-methyl modification, or 2'-fluoro modification or 2'-methoxyethyl (MOE) modification. As a phosphorothioate-type nucleic acid, a phosphodiester bond in the backbone of a nucleic acid may be replaced with a phosphorothioate bond. Artificial nucleic acids include nucleic acids in which the oxygen atom at position 2' and the carbon atom at position 4' are bridged. Such artificial nucleic acids include: locked nucleic acid (LNA), which is a bridged DNA in which the oxygen atom at position 2' and the carbon atom at position 4' are bridged via methylene; ENA, in which the oxygen atom at position 2' and the carbon atom at position 4' are bridged via ethylene; bridged nucleic acid (BNA) such as BNACOC, in which the oxygen atom at position 2' and the carbon atom at position 4' are bridged via -CH₂OCH₂-, and BNANC, in which the oxygen atom at position 2' and the carbon atom at position 4' are bridged via -NR-CH₂- {wherein R is methyl or a hydrogen atom}; cMOE, in which the oxygen atom at position 2' and the carbon atom at position 4' are bridged via -CH₂(OCH₃)-; cEt, in which the oxygen atom at position 2' and the carbon atom at position 4' are bridged via -CH₂(CH₃)-; AmNA, in which the carbon atoms at position 2' and position 4' are bridged via amide; scpBNA, in which the oxygen atom at position 2' and the carbon atom at position 4' are bridged via methylene and cyclopropane is formed at position 6'; and peptide nucleic acid (PNA), in which a polymer in which molecules of N-(2-aminoethyl)glycine, instead of deoxyribose or ribose, are amide-bonded serves as the main chain. Examples of RNA include artificial RNA for gene silencing such as siRNA and shRNA, non-coding RNA such as microRNA (miRNA) and aptamer, and natural RNA such as mRNA. These RNAs can be modified for stabilization in living bodies.

Examples of proteins encoded by mRNA include a component specific to a heterologous organism such as a pathogen or a part of the component, and a component specific to cancer cells or a part of the component. Examples of the pathogen include pathogenic viruses, pathogenic microorganisms, and pathogenic parasites. The pathogenic viruses include viruses selected from the group consisting of human immunodeficiency viruses (HIV), hepatitis A, hepatitis B, hepatitis C, herpesviruses, adenoviruses, influenza viruses, flaviviruses, echoviruses, rhinoviruses, coxsackieviruses, coronaviruses (e.g., severe acute respiratory syndrome-associated coronavirus (SARS-CoV), middle east respiratory syndrome coronavirus (MERS), SARS-CoV-2), respiratory syncytial viruses, mumps viruses, rotaviruses, measles viruses, rubella viruses, parvoviruses, vaccinia viruses, human T-cell leukemia (HTL) viruses, dengue viruses, papillomaviruses, molluscum viruses, polioviruses, rabies viruses, John Cunningham (JC) viruses, and arboviral encephalitis viruses. The pathogenic microorganisms include pathogenic bacteria, and the pathogenic bacteria include bacteria selected from the group consisting of chlamydiae, bacteria belonging to the order Rickettsiales, mycobacteria, staphylococci, streptococci, pneumococci, meningococci and gonococci, the genus Klebsiella, the genus Proteus, the genus Serratia, the genus Pseudomonas, the genus Legionella, diphtheria, the genus Salmonella, bacilli, cholera, tetanus, botulism, anthraces, plague, leptospirosis, and Lyme disease bacteria. The pathogenic microorganisms include pathogenic fungi, and the pathogenic fungi include pathogenic fungi selected from the group consisting of Candida (albicans, krusei, glabrata, tropicalis, and so on), Cryptococcus neoformans, Aspergillus (fumigatus, niger, and so on), the order Mucorales (the genus Mucor, the genus Absidia, the genus Rhizopus), Sporothrix schenckii, Blastomyces dermatitidis, Paracoccidioides brasiliensis, Coccidioides immitis, and Histoplasma capsulatum. The pathogenic parasites include pathogenic parasites selected from the group consisting of Entamoeba histolytica, Balantidium coli, Naegleria fowleri, the genus Acanthamoeba, Giardia lamblia, the genus Cryptosporidium, Pneumocystis carinii, Plasmodium vivax, Babesia microti, Trypanosoma brucei, Trypanosoma cruzi, Leishmania donovani, Toxoplasma gondii, and Ancylostoma braziliense. Accordingly, in an embodiment of the present invention, the mRNA can encode a constituent component (preferably a surface antigen) of any of those pathogens or a part of the constituent component. Among surface antigens of pathogens, in particular, an antigen that is used by a pathogen for infection of cells, such as S protein of betacoronaviruses, can be preferably encoded by the mRNA in the present invention. Examples of cancer include cancers selected from the group consisting of bladder cancer, breast cancer, uterine cancer, endometrial cancer, ovarian cancer, colorectal cancer, colon cancer, head and neck cancer, lung cancer, gastric cancer, germ cell cancer, bone cancer, squamous epithelial cell cancer, skin cancer, central nervous system neoplasm, lymphoma, leukemia, sarcoma, virus-associated cancer, small cell lung cancer, non-small cell lung cancer, gastrointestinal cancer, Hodgkin's or non-Hodgkin's lymphoma, pancreatic cancer, glioblastoma, glioma, cervical cancer, ovarian cancer, liver cancer, myeloma, salivary gland cancer, kidney cancer, basal cell cancer, melanoma, prostate cancer, vulvar cancer, thyroid cancer, testis cancer, esophageal cancer, and head and neck cancer. In an embodiment of the present invention, the mRNA can be an antigen specific to any of those cancers (a cancer-specific antigen, preferably a cancer-specific surface antigen) or a part of the antigen. The cancer-specific antigen can be, for example, a neoantigen or a neoantigen-specific part thereof. Examples of the cancer-specific antigen include cancer-testis antigens (e.g., proteins of the MAGE family, NY-ESO-1), differentiation antigens (e.g., tyrosine kinase of melanoma, Melan-A/MART-1, PSA of prostate cancer), overexpressed cancer antigens (e.g., Her-2/Neu, survivin, telomerase, and WT-1), cancer-inducing mutants of β-catenin, cancer-inducing mutants of CDK4, MUC-1, in particular, MUC-1 having an altered glycosylation pattern, and E6 or E7 of human papillomaviruses. Regarding these, those skilled in the art could appropriately select an antigen to prepare an mRNA suitable for the present invention according to the type of an infection affecting a subject, the type of cancer and the type of an antigen expressed in the cancer. Even an mRNA for a naturally mutated protein can be appropriately designed.

There is no limitation to surfactants as long as they are pharmaceutically acceptable. For example, ionic surfactants such as anionic (negative-ionic) surfactants and cationic (positive-ionic) surfactants are preferably used as such surfactants. Anionic (negative-ionic) surfactants and cationic (positive-ionic) surfactants are preferred in terms of superiority in the ability to form complexes with molecule of interest (e.g., a nucleic acid). In particular, a mixture of a cationic surfactant and an anionic surfactant is used as a surfactant in the present disclosure.

The surfactant is preferably a lipophilic ionic surfactant having an HLB (Hydrophile-Lipophile Balance) value of 10 or less because complexes can be dispersed in an oil phase with ease. The HLB of the ionic surfactant is preferably 8 or less, more preferably 5 or less, and particularly preferably 3 or less. The HLB of the ionic surfactant can be, for example, 1 to 3, 3 to 5, or 5 to 10. In an embodiment, the HLB of the ionic surfactant can 5 to 10. In an embodiment, the HLB of the cationic surfactant can be 1 to 10, for example, 1 to 3, 3 to 5, or 5 to 10. In an embodiment, the HLB of the cationic surfactant can be 5 to 10.

The ratio of the mass of the surfactant to the mass of a molecule of interest (e.g., a nucleic acid) in complex (parts by mass of the surfactant to 1 part by mass of a molecule of interest (e.g., a nucleic acid)) can be, for example, 1 to 200, preferably 20 to 180, and more preferably 20 to 100. In the case where the nucleic acid is antisense oligo, the mass ratio can be preferably 30 to 50; in the case where the nucleic acid is an mRNA, the mass ratio can be preferably 50 to 100.

For the amount of complexes contained in the composition or percutaneous absorption agent according to the present embodiment, any concentration that allows dispersion is permitted without limitation. The amount of complexes contained in the composition or percutaneous absorption agent is, for example, 0.6 w/v% to 60 w/v%. The amount of molecule of interest (e.g., a nucleic acid) contained in the percutaneous absorption agent is not limited, and can be, for example, 0.1 to 10 mg/mL, 0.2 to 8 mg/mL, 0.3 to 7 mg/mL, or preferably 1 to 5 mg/mL.

The complex may consist only of a molecule of interest (e.g., a nucleic acid) and a surfactant, or contain an additional component such as a stabilizing agent. The stabilizing agent is, for example, a hydrophilic protein and a polysaccharide, preferably with a molecular weight of 10,000 or more. The stabilizing agent together with a molecule of interest (e.g., a nucleic acid) is covered with the surfactant, thereby enhancing the stability of the complex and successfully preventing the leakage or the like of the molecule of interest (e.g., a nucleic acid) to the outside of the complex in the composition or percutaneous absorption agent.

Examples of the protein include serum albumin, ovalbumin, casein, lysozyme, and lipase. One of these proteins may be used alone, and two or more thereof may be used in combination.

Examples of the polysaccharide include LM pectin, HM pectin, hydroxypropylmethylcellulose phthalate, heparin, alginic acid, and carboxymethylcellulose. One of these polysaccharides may be used alone, and two or more thereof may be used in combination.

The ratio of the mass of the stabilizing agent to the mass of a molecule of interest (e.g., a nucleic acid) in each complex is preferably 0.01 to 100, more preferably 0.1 to 10, and even more preferably 0.5 to 5.

The oil phase may be in the form of liquid, or in the form of solid with fluidity. The base material of the oil phase may be any oily base material acceptable for use in pharmaceutical formulations, preferably acceptable for use in pharmaceutical formulations for transdermal delivery, without limitation. Either an oil in the form of liquid at normal temperature (25°C) or an oil in the form of solid at normal temperature can be used. Even the origin of the raw material is not limited; for example, any of natural substances and synthetic substances can be used. The oil phase may be vegetable oil such as soybean oil, cottonseed oil, rapeseed oil, sesame oil, corn oil, peanut oil, safflower oil, sunflower oil, olive oil, canola oil, Perilla seed oil, fennel oil, cacao oil, cinnamon oil, Mentha oil, and bergamot oil, or animal oil such as beef tallow, pork oil, and fish oil. The oil phase may be a neutral lipid such as glyceride, triolein, trilinolein, tripalmitin, tristearin, trimyristin, and triarachidonin, or a synthetic lipid.

The oil phase may be a sterol derivative such as cholesteryl oleate, cholesteryl linoleate, cholesteryl myristate, cholesteryl palmidate, and cholesleryl arachidonate, or a long-chain fatty acid ester such as isopropyl myristate (IPM), octyldodecyl myristate, cetyl myristate, ethyl oleate, ethyl linoleate, isopropyl linoleate, isopropyl palmitate, and butyl stearate. Alternatively, the oil phase may be a carboxylate ester such as ethyl lactate, cetyl lactate, triethyl citrate, diisopropyl adipate, diethyl sebacate, diisopropyl sebacate, and cetyl 2-ethylhexanoate, or a hydrocarbon such as petrolatum, paraffin squalane, and vegetable squalane, or a silicone. One of the oily base materials may be used alone, and two or more thereof may be used as a mixture.

For the oil phase, triglyceride or a vegetable oil containing triglyceride as a main component can be used, and soybean oil is preferred in a practical sense, and highly purified soybean oil is more preferred. A neutral lipid or a long-chain fatty acid ester is also preferably used, and a long-chain fatty acid ester is more preferred, and IPM is even more preferred.

The amount of the oil phase contained in the composition or percutaneous absorption agent according to the present embodiment depends on, for example, the type of the oil component and other constituent components, and is preferably 50 w/v% to 99.5 w/v%, and more preferably 60 w/v% to 90 w/v%.

The term ultrapure water herein refers to water satisfying the standard of ASTM D 5196-06 (2018), and the standard of ASTM D 5196-06 (2018) specifies ultrapure water as water having a total amount of inorganic ions (in particular, aluminum, ammonia, arsenic, cadmium, chromium, fluoride ion, cobalt, copper, iron, lead, magnesium, nickel, potassium, sodium, titanium, and zinc, and chloride ion, nitrate ion, phosphate ion, and sulfate ion) of 1 µg/L or a specific resistance of 18.2 MΩcm at 25°C, a total organic carbon content of 20 ppb or less, a bacterial colony count of 100 cfu/100 mL or less, and an endotoxin level of 0.01 EU/mL or less. For ultrapure water for a solution containing a nucleic acid, nucleases are required to be below the detection limit. For ultrapure water for a solution containing a protein, proteases are required to be below the detection limit.

Various cations can be used as the cation, and, for example, cationic lipids can be used. Various anions can be used as the anion, and, for example, anionic lipids can be used. The cationic lipids and anionic lipids are as described above. Specifically, a cationic lipid contains, for example, a cation moiety and an aliphatic chain (e.g., a long-chain aliphatic chain) such as alkyl, alkenyl, or alkynyl.

The ionic liquid is a salt that consists of a cation and an anion and is present as a liquid in a wide temperature range. In the present invention, the ionic liquid is liquid at environmental temperature (e.g., room temperature, for example, 25°C). For example, the anion is preferably a carboxylate ion resulting from the dissociation of proton from the carboxy group of a fatty acid, or a derivative of the carboxylate ion. The fatty acid to generate a carboxylate ion may be a saturated fatty acid or an unsaturated fatty acid. Examples of the saturated fatty acid can include myristic acid, palmitic acid, stearic acid, and lauric acid, and examples of the unsaturated fatty acid can include oleic acid, linoleic acid, α-linolenic acid, γ-linolenic acid, arachidonic acid, icosapentaenoic acid, docosahexaenoic acid, and erucic acid.

The cation that can form the ionic liquid is a cationic lipid, and preferably a phospholipid having a cationic group. Here, the term "phospholipid" refers to a lipid containing a phosphate ester structure, and the term "cationic group" refers to a substituent charged with positive electricity. The two hydroxy groups of a phosphate group are each substituted with a lower alkyl (e.g., ethyl) to lose the charges, and any of the lower alkyls is substituted with an amine (a primary amine, a secondary amine, or a tertiary amine) to give a cationic lipid. Moreover, the cation that can form the ionic liquid is preferably a glycerophospholipid having a cationic group, and more preferably a derivative of phosphatidylcholine. It is preferable that the anion that can form the ionic liquid be linoleic acid and the cation that can form the ionic liquid be 1,2-dimyristoyl-sn-glycero-3-ethylphosphocholine. Among lipids, unsaturated lipids have higher tendencies to form an ionic liquid than saturated lipids.

The amount of a skin permeation promoter contained in the composition or percutaneous absorption agent according to the present embodiment is determined from the viewpoint of enhancing the skin permeation of molecule of interest (e.g., a nucleic acid), and is, for example, 1 to 20% by mass, 2 to 18% by mass, 3 to 16% by mass, 4 to 15% by mass, or 5 to 10% by mass.

### (Preparation of formulation with emulsion method)

The composition or percutaneous absorption agent according to the present embodiment can be produced, for example, with a method including an emulsion preparation step, a complex preparation step, and a dispersing step. In the emulsion preparation step, molecule of interest (e.g., a nucleic acid), a surfactant, and an organic solvent solution are mixed to prepare an emulsion of W/O type. The emulsion preparation step is performed under conditions suitable for the formation of the W/O emulsion. In the emulsion preparation step, preferably, an aqueous solution containing molecule of interest (e.g., a nucleic acid) (the aqueous solution may further contain a cation) and an organic solvent solution containing a surfactant are mixed together, and the mixture is emulsified and dispersed to prepare an emulsion of W/O type. The organic solvent solution is preferably a volatile organic solvent solution for easy removal of the organic solvent solution in a later drying step.

The concentration of molecule of interest (e.g., a nucleic acid) in the aqueous solution of molecule of interest (e.g., a nucleic acid) is not limited as long as the molecule of interest is substantially completely dissolved, and is, for example, about 0.1 mg/mL to about 30 mg/mL, about 0.4 mg/mL to about 30 mg/mL, or about 1 mg/mL to about 30 mg/mL. As necessary, a stabilizing agent may be added to the aqueous solution. The organic solvent may be any solvent that can dissolve the surfactant and be removed in the subsequent steps, without limitation. Examples of the organic solvent include aliphatic hydrocarbons such as hexane and cyclohexane and aromatic hydrocarbons such as toluene. The concentration of the surfactant in the organic solvent solution is not limited, and is, for example, 1 mg/mL to 100 mg/mL, 20 mg/mL to 90 mg/mL, or 40 mg/mL to 80 mg/mL.

Emulsifying and dispersing can be performed, for example, by using any of a stirrer, a high-speed shear stirrer, a colloid mill, a homogenizer, a flow-jet mixer, an ultrasonic emulsifying machine, and a vacuum emulsifying machine. The particle sizes of dispersed droplets can be controlled by adjusting the stirring intensity.

In the complex preparation step, the emulsion of W/O type obtained in the emulsion preparation step is dried to prepare molecule of interest (e.g., a nucleic acid)-surfactant complexes (solid complexes or solid particles). Any drying method is applicable without limitation, examples thereof include freeze-drying and vacuum drying, and freeze-drying is preferred. Moisture may cause the leakage of molecule of interest (e.g., a nucleic acid) in the formulation. The organic solvent may have adverse effects on living bodies. Therefore, it is preferable in the drying step to substantially completely remove moisture and the organic solvent. For example, it is appropriate to set the moisture content to about 1% by mass or less, 0.9% by mass or less, 0.8% by mass or less, 0.7% by mass or less, 0.6% by mass or less, 0.5% by mass or less, 0.4% by mass or less, 0.3% by mass or less, 0.2% by mass or less, or 0.1% by mass or less, as measured with a known method. The complexes to be obtained in that manner, containing a molecule of interest (e.g., a nucleic acid) and a surfactant, can be solid complexes or solid particles having low moisture contents. In the case where the organic solvent is volatile, the organic solvent is naturally removed in the drying step.

In the dispersing step, the molecule of interest (e.g., a nucleic acid)-surfactant complexes (dispersoid) obtained in the complex preparation step are dispersed in an oil phase (dispersion medium). For dispersing the molecule of interest (e.g., a nucleic acid)-surfactant complexes, specifically, any of the methods shown as examples of techniques for emulsifying and dispersing in the emulsion preparation step can be used. Although depending on the compatibility or the like between the surfactant and the type of the oil phase, the amount of the oil phase to be used in the dispersing step is, for example, 1 mL to 200 mL per g of the molecule of interest (e.g., a nucleic acid)-surfactant complexes. As necessary, an additional component such as a skin permeation promoter can be dispersed in the oil phase. In the dispersing step, the composition or percutaneous absorption agent according to the present embodiment is obtained as a dispersion formulation or a suspension.

### (Preparation of formulation by ethanol dilution method)

The formulation of the present invention is also obtainable by an ethanol dilution method. In the ethanol dilution method, a composition containing a cation and an anion or a composition consisting of a cation and an anion can be used. The cation and the anion can be a cationic surfactant and an anionic surfactant, respectively. The principle of the ethanol dilution method is based on the fact that when ethanol (a lower alcohol is also applicable) containing water-soluble molecules having solubility in ethanol is diluted with water, molecules that cannot keep dissolution any more form complexes. Accordingly, the ethanol dilution method causes the formation of complexes containing a cation, an anion, and a molecule of interest (e.g., a nucleic acid) by mixing a lower alcohol solution containing a cation and an anion and an aqueous solution containing a pharmaceutical agent such as molecule of interest (e.g., a nucleic acid). The resulting complexes (dispersed in a low-concentration ethanol solution) can be directly used, or freeze-dried and dispersed in an oil phase for use. The resulting complexes (dispersed in a low-concentration ethanol solution) can be used, for example, for transfection of cells with molecule of interest (e.g., a nucleic acid) by bringing the complexes into contact with the cells. The complexes freeze-dried and dispersed in an oil phase can be used for transdermal administration.

The lower alcohol is an alcohol having six or less, preferably five or less, more preferably four or less, three or less, or two or less carbon atoms. Examples of the lower alcohol include methanol, ethanol, 1-propanol, isopropanol, 1-butanol, isobutyl alcohol, 2-butanol, and tert-butyl alcohol. For the lower alcohol, ethanol can be preferably used.

Accordingly, the present invention provides a composition for use in formation of a complex with a pharmaceutical ingredient, the composition containing a cation and an anion. In an embodiment, the composition can be an ionic liquid. Alternatively, the composition may further contain a lower alcohol in addition to the cation and the anion. In an embodiment, the lower alcohol can be ethanol. For the cation and the anion, those for use in forming the solid complexes can be used. The cation can be preferably a cationic lipid. The anion can be preferably an anionic lipid. The cation and the anion can be a cationic lipid and an anionic lipid, respectively. The cationic lipid and the anionic lipid each function as a surfactant, and hence the terms are used as those synonymous with a cationic surfactant and an anionic surfactant, respectively. In an embodiment, the composition contains or consists of a cationic surfactant, an anionic surfactant, and a lower alcohol (preferably ethanol).

When the composition containing or consisting of a cationic surfactant, an anionic surfactant, and a lower alcohol (preferably ethanol) and an aqueous solution containing molecule of interest (e.g., a nucleic acid) are mixed together, a lower alcohol aqueous solution containing complexes containing the cationic surfactant, the anionic surfactant, and a molecule of interest (e.g., a nucleic acid) instantaneously results. The resulting complexes include particles having particle sizes of about 5 nm to about 20 nm. The resulting complexes include aggregates each containing a plurality of particles having particle sizes of about 5 nm to about 20 nm. Aggregate can have, for example, a particle size of about 100 nm to about 500 nm, or about 100 to about 300 nm. In the case where the lower alcohol is an alcohol having low cytotoxicity such as ethanol or propanol, for example, molecule of interest (e.g., a nucleic acid) can be introduced into cells or cells can be transfected with molecule of interest (e.g., a nucleic acid) by bringing the resulting lower alcohol solution into direct contact with the cells. The resulting solution can be removed of the alcohol through processing such as dialysis. Use of an aqueous solution (e.g., physiological saline) as the external dialysis solution will give an aqueous solution (e.g., substantially free of any alcohol) containing the complexes.

The resulting lower alcohol solution can be freeze-dried without undergoing the emulsion-forming step and the resulting freeze-dried product can be then dispersed in an oil phase. As a result, a composition is obtained, the composition containing: solid complexes containing a cationic surfactant, an anionic surfactant, and a molecule of interest (e.g., a nucleic acid); and an oil phase, wherein the complexes are dispersed in the oil phase. Alternatively, by dispersing the resulting freeze-dried product in an aqueous phase, a composition is obtained, the composition containing: complexes or solid complexes each containing a cationic surfactant, an anionic surfactant, and a molecule of interest (e.g., a nucleic acid); and an aqueous phase, wherein the complexes are dispersed in the aqueous phase.

In place of the lower alcohol in the above description, an organic solvent such as acetone and acetonitrile may be used. In an embodiment, a lower alcohol is preferably used in the ethanol dilution method, and ethanol is preferably used. In an embodiment, acetone can be used. In an embodiment, acetonitrile can be used.

The resulting composition can be used as a percutaneous absorption agent. Alternatively, the resulting composition can be used as a composition for transdermal administration. The resulting composition may further contain an anionic surfactant. As described later, anionic surfactants (in particular, fatty acids) can attenuate the skin barrier function. Therefore, further addition of an anionic surfactant can enhance the characteristics of the composition as a percutaneous absorption agent. Molecule of interest (e.g., a nucleic acid) can be allowed to permeate into skin by applying the resulting composition to skin. The resulting composition containing complexes and an aqueous phase can be used for introduction of molecule of interest (e.g., a nucleic acid) into cells or transfection of cells with molecule of interest (e.g., a nucleic acid).

The nucleic acid in the above description can be replaced with a bioactive substance such as a protein and a low-molecular-weight compound. This allows effective transdermal administration of a bioactive substance such as a protein and a low-molecular-weight compound in place of a nucleic acid.

In the complexes each containing a cationic surfactant, an anionic surfactant, and a molecule of interest (e.g., a nucleic acid), the cationic surfactant and the anionic surfactant are contained at a ratio of 50 mol% or more, 60 mol% or more, 70 mol% or more, 80 mol% or more, 90 mol% or more, 95 mol% or more, 98 mol% or more, 99 mol% or more, or 100 mol% to the contained surfactants. Preferably, the cationic surfactant and the anionic surfactant are contained in each complex at 1:0.5 to 1:2, 1:0.6 to 1:1.5, 1:0.8 to 1:1.25, 1:0.9 to 1:1.11, or about 1:about 1. In an embodiment, the cationic surfactant and the anionic surfactant are electrically neutralized in each complex.

### (Composition or percutaneous absorption agent)

The composition or percutaneous absorption agent according to the present embodiment may be formulated with addition of an additional supplementary component. Examples of the additional supplementary component include excipients, coloring agents, lubricants, binders, emulsifying agents, thickeners, wetting agents, stabilizers, preservatives, solvents, solubilizers, suspending agents, buffering agents, pH adjusters, and bases, and these can be blended with normal blend ratios.

Examples of the excipients include saccharides such as white soft sugar, starch derivatives such as dextrin, cellulose derivatives such as carmellose sodium, and water-soluble polymers such as xanthan gum. Examples of the lubricants can include metal stearates such as calcium stearate and magnesium stearate, lauryl sulfates, and the starch derivatives for the aforementioned excipients. Examples of the binders include the aforementioned excipients and macrogol. The wetting agents are glycerin and the like. The stabilizers are methylparaben, para-hydroxybenzoates, alcohols such as chlorobutanol, benzyl alcohol, and phenylethyl alcohol, benzalkonium chloride, phenols such as phenol and cresol, thimerosal, acetic anhydride, sorbic acid, and the like. Examples of the solvents include water and ethanol/glycerin. The suspending agents are carmellose sodium and the like. Examples of the bases include polyethylene glycol, crotamiton, diethyl sebacate, and petrolatum.

A dispersion or suspension obtained by the above production method may be directly used, and can be mixed with an additional supplementary component, as necessary, and processed into a topical agent such as a patch, an ointment, a lotion, an aerosol, a cataplasm, an aqueous gel patch, a cream, a gel, a plaster, a reservoir-type patch, a matrix-type patch, and a tape with a known method. Thus, a percutaneous absorption formulation can be obtained.

The dose of the composition or percutaneous absorption agent according to the present embodiment can be adjusted to fit to the age of the patient, the types of symptoms and adaptation disease, and so on. For example, administration can be performed for several weeks to several months in such a manner that the amount of molecule of interest (e.g., a nucleic acid) reaches 1 µg to 30 mg, preferably 10 µg to 10 mg, more preferably 30 µg to 3 mg per administration for an adult in typical cases.

Vertebrates are preferred, and mammals are more preferred as subjects to which the composition or percutaneous absorption agent according to the present embodiment is applied. Examples of the mammals include: humans, chimpanzees and other primates, and domestic animals, pet animals, and experimental animals such as dogs, cats, rabbits, horses, sheep, goats, bovines, pigs, rats, mice, and guinea pigs. Humans are particularly preferable as such mammals. The composition or percutaneous absorption agent according to the present embodiment can be transdermally administered to a subject, or applied to the skin of a subject.

The composition or percutaneous absorption agent according to the present embodiment, in which solid molecule of interest (e.g., a nucleic acid)-surfactant complexes are dispersed in an oil phase, allows molecule of interest (e.g., a nucleic acid) that hardly permeate into skin to permeate from skin into a body as demonstrated in Examples shown later.

The composition or percutaneous absorption agent according to the present embodiment can be used for diseases that can be treated with a nucleic acid or a nucleic acid drug. Such a disease is a disease that can be treated or prevented or the progression of which can be stopped or inhibited by targeting RNA, in particular. Examples of the diseases include skin diseases such as skin carcinoma, rheumatoid arthritis, and atopic dermatitis. The composition or percutaneous absorption agent according to the present embodiment is also suitable as a transdermal DDS for RNA vaccines.

Provided as another embodiment is a method for treating any of the diseases, the method including using the composition or percutaneous absorption agent containing a therapeutically effective amount of molecule of interest **(e.g.,** a nucleic acid) for a subject in need of treatment. The therapeutically effective amount of molecule of interest (e.g., a nucleic acid) is an amount shown as the dose of molecule of interest (e.g., a nucleic acid) for the composition or percutaneous absorption agent, wherein the dose has been set to fit the disease. Provided as still another embodiment is the composition or percutaneous absorption agent for treatment of any of the diseases.

Provided as another embodiment is a method for enhancing the transdermal absorption of molecule of interest (e.g., a nucleic acid). The method includes forming a topical agent by covering molecule of interest (e.g., a nucleic acid) with a surfactant to form solid molecule of interest (e.g., a nucleic acid)-surfactant complexes and dispersing the complexes in an oil phase. The method enables the enhancement of the transdermal absorption of molecule of interest (e.g., a nucleic acid) that are difficult for use as a topical agent because of low transdermal absorption and are incapable of exerting sufficient effects in a topical agent.

Instead of the nucleic acid described herein, a drug other than nucleic acids can be used in the present invention. Examples of the drug other than nucleic acids include a protein and a compound (preferably a hydrophilic protein and a hydrophilic compound). The protein can be, for example, a protein (preferably a hydrophilic protein, more preferably a cationic hydrophilic protein) having a molecular weight of 5 kDa to 100 kDa, but is not limited thereto. The compound can be, for example, a compound (preferably a hydrophilic compound, more preferably a cationic hydrophilic compound) having a molecular weight of 800 to 10,000.

Examples of the protein include bioactive substances such as cytokines (e.g., interleukin-1 (IL-1), IL-2, IL-3, IL-4, IL-5, IL-6, IL-7, IL-8, IL-9, IL-10, IL-11, IL-12, IL-13, IL-14, IL-15, α-interferon, β-interferon, γ-interferon, angiostatin, thrombospondin, endostatin, GM-CSF, G-CSF, M-CSF, tumor necrosis factor). Other examples of the protein include agalsidase beta, laronidase, alteplase, habinafusp alfa, urokinase, idursulfase, sebelipase alfa, monteplase, asfotase alfa, imiglucerase, velaglucerase, agalsidase alfa, alglucosidase alfa, avalglucosidase alfa, idursulfase, galsulfase, elosulfase alfa, rasburicase, dornase, alfa, asfotase alfa, collagenase, sebelipase, condoliase, elapegademase, cerliponase, alfa, glucarpidase, vestronidase alfa, olipudase alfa, blood coagulation factors (e.g., I, II, III, IV, V, VI, VII, VIII, IX, X, XI, and XII), insulin, somatropin, erythropoietin, and chemokine. Still other examples of the protein include Cas9 protein, RNA polymerases, transcription factors, ribosome, cyclin-dependent kinases, growth factors (e.g., epidermal growth factors (EGFs), platelet-derived growth factors (PDGFs), fibroblast growth factors (FGFs), insulin-like growth factors (IGFs), nerve growth factors (NGFs), vascular endothelial growth factors (VEGFs), hepatocyte growth factors (HGFs), transforming growth factors (TGFs), colony-stimulating factors (CSFs), erythropoietin (EPO), granulocyte colony-stimulating factors (G-CSFs), granulocyte-macrophage colony-stimulating factors (GM-CSFs), macrophage colony-stimulating factors (M-CSFs), interleukins (some interleukins have growth factor activity), bone morphogenetic proteins (BMPs), growth differentiation factors (GDFs), glial cell line-derived neurotrophic factors (GDNFs), gangliocyte neurotrophic factors (CNTFs), brain-derived neurotrophic factors (BDNFs), neurotrophins (NTs), keratinocyte growth factors (KGFs), and thrombopoietin (TPO)). Still other examples of the protein include serum albumin, ovalbumin, casein, lysozyme, and lipase. One of those proteins may be used alone, and two or more thereof may be used in combination. An example of the protein is a protein complex.

Examples of the nucleic acid include a DNA or an RNA (e.g., an mRNA). Examples of proteins encoded by the nucleic acid include a component specific to a heterologous organism such as a pathogen or a part of the component, and a component specific to cancer cells or a part of the component. Examples of the pathogen include pathogenic viruses, pathogenic microorganisms, and pathogenic parasites. The pathogenic viruses include viruses selected from the group consisting of human immunodeficiency viruses (HIV), hepatitis A, hepatitis B, hepatitis C, herpesviruses, adenoviruses, influenza viruses, flaviviruses, echoviruses, rhinoviruses, coxsackieviruses, coronaviruses (e.g., severe acute respiratory syndrome-associated coronavirus (SARS-CoV), middle east respiratory syndrome coronavirus (MERS), SARS-CoV-2), respiratory syncytial viruses, mumps viruses, rotaviruses, measles viruses, rubella viruses, parvoviruses, vaccinia viruses, human T-cell leukemia (HTL) viruses, dengue viruses, papillomaviruses, molluscum viruses, polioviruses, rabies viruses, John Cunningham (JC) viruses, and arboviral encephalitis viruses. The pathogenic microorganisms include pathogenic bacteria, and the pathogenic bacteria include bacteria selected from the group consisting of chlamydiae, bacteria belonging to the order Rickettsiales, mycobacteria, staphylococci, streptococci, pneumococci, meningococci and gonococci, the genus Klebsiella, the genus Proteus, the genus Serratia, the genus Pseudomonas, the genus Legionella, diphtheria, the genus Salmonella, bacilli, cholera, tetanus, botulism, anthraces, plague, leptospirosis, and Lyme disease bacteria. The pathogenic microorganisms include pathogenic fungi, and the pathogenic fungi include pathogenic fungi selected from the group consisting of Candida (albicans, krusei, glabrata, tropicalis, and so on), Cryptococcus neoformans, Aspergillus (fumigatus, niger, and so on), the order Mucorales (the genus Mucor, the genus Absidia, the genus Rhizopus), Sporothrix schenckii, Blastomyces dermatitidis, Paracoccidioides brasiliensis, Coccidioides immitis, and Histoplasma capsulatum. The pathogenic parasites include pathogenic parasites selected from the group consisting of Entamoeba histolytica, Balantidium coli, Naegleria fowleri, the genus Acanthamoeba, Giardia lamblia, the genus Cryptosporidium, Pneumocystis carinii, Plasmodium vivax, Babesia microti, Trypanosoma brucei, Trypanosoma cruzi, Leishmania donovani, Toxoplasma gondii, and Ancylostoma braziliense. Accordingly, in an embodiment of the present invention, the mRNA can encode a constituent component (preferably a surface antigen) of any of those pathogens or a part of the constituent component. Among surface antigens of pathogens, in particular, an antigen that is used by a pathogen for infection of cells, such as S protein of betacoronaviruses, can be preferably encoded by the mRNA in the present invention. Examples of cancer include cancers selected from the group consisting of bladder cancer, breast cancer, uterine cancer, endometrial cancer, ovarian cancer, colorectal cancer, colon cancer, head and neck cancer, lung cancer, gastric cancer, germ cell cancer, bone cancer, squamous epithelial cell cancer, skin cancer, central nervous system neoplasm, lymphoma, leukemia, sarcoma, virus-associated cancer, small cell lung cancer, non-small cell lung cancer, gastrointestinal cancer, Hodgkin's or non-Hodgkin's lymphoma, pancreatic cancer, glioblastoma, glioma, cervical cancer, ovarian cancer, liver cancer, myeloma, salivary gland cancer, kidney cancer, basal cell cancer, melanoma, prostate cancer, vulvar cancer, thyroid cancer, testis cancer, esophageal cancer, and head and neck cancer. In an embodiment of the present invention, the mRNA can be an antigen specific to any of those cancers (a cancer-specific antigen, preferably a cancer-specific surface antigen) or a part of the antigen. The cancer-specific antigen can be, for example, a neoantigen or a neoantigen-specific part thereof. Examples of the cancer-specific antigen include cancer-testis antigens (e.g., proteins of the MAGE family, NY-ESO-1), differentiation antigens (e.g., tyrosine kinase of melanoma, Melan-A/MART-1, PSA of prostate cancer), overexpressed cancer antigens (e.g., Her-2/Neu, survivin, telomerase, and WT-1), cancer-inducing mutants of β-catenin, cancer-inducing mutants of CDK4, MUC-1, in particular, MUC-1 having an altered glycosylation pattern, and E6 or E7 of human papillomaviruses. Regarding these, those skilled in the art could appropriately select an antigen to prepare an mRNA suitable for the present invention according to the type of an infection affecting a subject, the type of cancer and the type of an antigen expressed in the cancer. The nucleic acid may be one encoding any of the proteins shown as examples. Even an mRNA for a natural mutated protein can be appropriately designed.

Examples of the nucleic acid include fomivirsen, pegaptanib, mipomersen, eteplirsen, nusimersen, CpG1018, inotersen, patisiran, volanesorsen, givosiran, golodirsen, viltolarsen, inclisiran, lumasiran, casimersen, vutrisiran, defibrotide, trabedersen, and tofersen. For the nucleic acid, various nucleic acids such as other Antisense oligo, siRNAs, shRNAs, and miRNAs can be used.

Examples of the molecule of interest include low-molecular-weight compounds. Examples of the low-molecular-weight compounds include antioxidants (e.g., ascorbic acid, isoascorbic acid, vitamin E, catechin, dibutylhydroxytoluene, tocopherol, butylhydroxyanisole, polyphenol, resveratrol (resveratrol 3,5,4'-trihydroxy-trans-stilbene), coenzyme Q10, N-acetylcysteine, 2,2,6,6-tetramethylpiperidine 1-oxyl (TEMPO), superoxide dismutase (SOD), nicotinamide mononucleotide (NMN), astaxanthin, β-carotene, and glutathione. Examples of the low-molecular-weight compounds include antitumor agents. Examples of the antitumor agents include alkylating agents, anthracycline, anti-hormonal agents, aromatase inhibitors, antiandrogens, protein kinase inhibitors, lipid kinase inhibitors, antisense oligonucleotides, ribozymes, antimetabolites, topoisomerase inhibitors, cytotoxic agents or antitumor antibiotics, proteasome inhibitors, anti-microtubule agents, EGFR antagonists, retinoids, tyrosine kinase inhibitors, histone deacetylase inhibitors, and combinations of any of them. Examples of the antitumor agents include erlotinib, bortezomib, disulfiram, epigallocatechin gallate, salinosporamide **A,** carfilzomib, 17-AAG (geldanamycin), radicicol, lactate dehydrogenase A (LDH-A), fulvestrant, sunitib, letrozole, imatinib mesylate, finasnate, oxaliplatin, 5-FU (5-fluorouracil), leucovorin, rapamycin, lapatinib, lonafarnib, sorafenib, gefitinib, AG1478, and alkylsulfonates such as busulfan, improsulfan, and piposulfan; aziridines such as benzodopa, carboquone, meturedopa, and uredopa; ethyleneimine and methylamelamine (including altretamine, triethylenemelamine, triethylenephosphorylamide, triethylenethiophosphorylamide); acetogenin (in particular, bullatacin and butallacinone); camptothecin (including topotecan and irinotecan); bryostatin; kallistatin; CC-1065 (including adozelesin, carzelesin, and bizelesin synthetic analogs thereof); cryptophycin (in particular, cryptophycin 1 and cryptophycin 8); adrenocorticosteroids (including prednisone and prednisolone); cyproterone acetate; 5α-reductases (including finasteride and dutasteride); vorinostat, romidepsin, panobinostat, valproic acid, mocetinostatdolastatin; aldesleukin, talc duocarmycin (including synthetic analogs, KW-2189 and CB1-TM1); eleutherobin; pancratistatin; sarcodictyin; spongistatin; antibiotics such as enediyne antibiotics (e.g., including calicheamicin, in particular, calicheamicin γ1I and calicheamicin ω1I (Angew Chem. Intl. Engl. ver. 1994 33: 183-186); dynemicin (including dynemicin A); bisphosphonates such as clodronate; esperamicin; and neocarzinostatin chromophores and related chromoprotein enediyne antibiotic chromophores), aclacinomycin, actinomycin, authramycin, azaserine, bleomycin, cactinomycin, carabicin, carminomycin, carzinophilin, chromomycinis, dactinomycin, daunorubicin, detorubicin, 6-diazo-5-oxo-L-norleucine, doxorubicin, morpholino-doxorubicin, cyanomorpholino-doxorubicin, 2-pyrrolino-doxorubicin, doxorubicin, and deoxydoxorubicin), epirubicin, esorubicin, idarubicin, marcellomycin, mitomycin such as mitomycin C, mycophenolic acid, nogalamycin, olivomycin, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folate analogs such as denopterin, methotrexate, pteropterin, and trimetrexate; purine analogs such as fludarabine, 6-mercaptopurine, thiamiprine, and thioguanine; pyrimidine analogs such as ancitabine, azacytidine, 6-azauridine, carmofur, cytarabine, dideoxyuridine, doxifluridine, enocitabine, floxuridine, and imatinib (2-phenylaminopyrimidine derivative); androgens such as calusterone, dromostanolone propionate, epitostanol, mepitiostane, and testolactone; adrenolytics such as aminoglutethimide, mitotane, and trilostane; folate fluid replenishers such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; epothilone; ethoglucid; gallium nitrate; hydroxyurea; lentinan; lonidainine; maytansinoids such as maytansine and ansamitocin; mitoguazone; mitoxantrone; mopidanmol; nitraerine; pentostatin; phenamet; pirarubicin; losoxantrone; 2-ethylhydrazide; procarbazine; razoxane; rhizoxin; sizofiran; spirogermanium; tenuazonic acid; triaziquone; 2,2',2"-trichlorotriethylamine; trichothecene (in particular, T-2 toxin, verracurin A, roridin A, and anguidine); urethane; vindesine; dacarbazine; mannomustine; mitobronitol; mitolactol; pipobroman; gacytosine; arabinoside ("Ara-C"); cyclophosphamide; thiotepa; taxoids, for example, paclitaxel, docetaxel, doxetaxel; chlorambucil; gemcitabine; 6-thioguanine; mercaptopurine; methotrexate; platinum analogs such as cisplatin and carboplatin; vinblastine; etoposide (VP-16); ifosfamide; mitoxantrone; vincristine; vinorelbine; novantrone; teniposide; edatraxate; daunomycin; aminopterin; capecitabine; ibandronate; CPT-11; the topoisomerase inhibitor RFS2000; difluoromethylornithine (DMFO); retinoids such as retinoic acid; and pharmaceutically acceptable salts, acids, and derivatives of any of them. Examples of the low-molecular-weight compounds include those having physiological activity.

The present invention provides a composition for use in attenuating skin barrier or skin barrier functions, the composition containing an anionic surfactant. The present invention provides an anionic surfactant for use in attenuating skin barrier or skin barrier functions, or use of the anionic surfactant. The present invention provides use of an anionic surfactant in production of a composition for use in attenuating skin barrier or skin barrier functions. The anionic surfactant in the description can be preferably an anionic long-chain lipid. The anionic long-chain lipid can be preferably a fatty acid. The fatty acid can be preferably a long-chain fatty acid, and more preferably a long-chain unsaturated fatty acid. Examples of the long-chain unsaturated fatty acid include a long-chain unsaturated fatty acid having one or more double bonds. The long-chain unsaturated fatty acid having one or more double bonds is preferably in the cis-form. The long-chain unsaturated fatty acid having one or more double bonds can be, for example, any of the fatty acids shown above as examples, and, for example, linoleic acid or oleic acid. In an embodiment, the composition for use in attenuating skin barrier or skin barrier functions, the composition containing an anionic surfactant, may contain a cation (e.g., a cationic lipid) in addition to a fatty acid. The composition for use in attenuating skin barrier or skin barrier functions, the composition containing an anionic surfactant, may contain a cation (e.g., a cationic lipid) and molecule of interest (e.g., a nucleic acid) in addition to a fatty acid.

The present invention provides a composition containing: solid complexes each containing a surfactant and a pharmaceutical agent; and an oil phase, wherein the oil phase contains an anionic long-chain lipid, and the solid complexes are dispersed in the oil phase. For the surfactant, one having an HLB value of 10 or less can be preferably used.

The surfactant can be preferably a nonionic surfactant. The nonionic surfactant is not limited, and examples thereof can include polyglycerin-condensed ricinoleate, decaglycerin ester, glycerin fatty acid ester, polyglycerin fatty acid ester, polyoxyethylene glycerin fatty acid ester, sorbitan fatty acid ester, polyoxyethylene sorbit fatty acid ester, polyoxyethylene castor oil-hydrogenated castor oil, and sucrose fatty acid ester (sucrose stearate, sucrose palmitate, sucrose myristate, sucrose oleate, sucrose laurate, sucrose erucate, sucrose mixed fatty acid ester). One selected from them or a mixture of two or more of them may be used. As the nonionic surfactant, an ester compound derived from an acid or an unsaturated fatty acid such as oleic acid as a raw material is preferred, and more preferred examples include sucrose erucate, sucrose oleate, and sucrose mixed fatty acid ester. Alternatively, one or two or more selected from the group consisting of glycerin fatty acid ester, polyglycerin fatty acid ester, polyoxyethylene glycerin fatty acid ester, sorbitan fatty acid ester, sucrose fatty acid ester, polyoxyethylene sorbit fatty acid ester, and polyoxyethylene castor oil and hydrogenated castor oil can be used.

The surfactant in the above description can be preferably a cationic surfactant. For the cationic surfactant, for example, any of the cationic surfactants shown as examples can be used.

The following more specifically describes the present invention with Examples, but the present invention is not limited by Examples.

### Examples

### Example 1: Preparation of ionic surfactant-nucleic acid complexes each composed of cationic surfactant, anionic surfactant, and bioactive substance

In the present example, nucleic acid-surfactant solid complexes each composed of a cationic surfactant, an anionic surfactant, and a nucleic acid were prepared. In the following, a mixture of EDMPC as a cationic surfactant and linoleic acid as an anionic surfactant ([EDMPC][Lin]) is used as a surfactant mixture, unless otherwise specified. This mixture exhibits the characteristics of ionic liquid at normal temperature.

1,2-Dimyristoyl-sn-glycero-3-ethyl-phosphatidylcholine (EDMPC) (WO2021/192861, the entire of which is incorporated herein by reference) was used as the cationic surfactant. Linoleic acid (Lin), oleic acid (Ole), or stearic acid (Ste) was used as the anionic surfactant. All of these cationic surfactant and anionic surfactants are biocompatible.

### [Formula 2]

| **Role** | **Name** | **Molecular structure** |
|---|---|---|
| Cation | 1,2-dimyristoyl-sn-glycero-3-eth yl-phosphatidylcholine (EDMPC) | 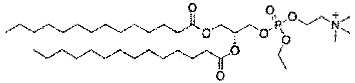 MW: 706.9 |
| Anion | Linoleic acid (Lin) | 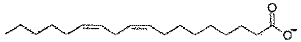 MW: 280.4 |
| | Oleic acid (Ole) | 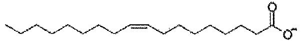 MW: 282.5 |
| | Stearic acid (Ste) | 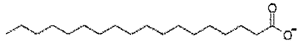 MW: 284.5 |
| Oil base | Isopropyl myristate (IPM) | 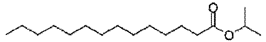 MW: 270.5 |
| Model nucleic acid | Trabedersen (ssDNA, 18 mer) | Sequence: |
| | | 5' -CGGCATGTCTATTTTGTA-3' |
| | | MW: 5770 |

One of an mRNA, an Antisense oligo, an embodimentl protein, and an embodimentl low-molecular-weight compound was used as the nucleic acid. The mRNA used was an mRNA containing an open reading frame (884mer) encoding green fluorescent protein in a translatable manner with a 5' cap and polyA.

### Reagents

The 1,2-dimyristoyl-sn-glycero-3-phosphatidylcholine (DMPC), linoleic acid (Lin), oleic acid (Ole), and stearic acid (Ste) were purchased from Tokyo Chemical Industry Co., Ltd., 5 mol/L hydrochloric acid, chloroform (super-dehydrated), and ammonium formate from KISHIDA CHEMICAL Co., Ltd., cyclohexane and acetonitrile from FUJIFILM Wako Pure Chemical Corporation, ER-290 and L-195 from Mitsubishi-Kagaku Foods Corporation, 4% paraformaldehyde from MUTO PURE CHEMICALS CO., LTD., and Trabedersen from Eurofins Genomics K.K.

### Experimental animals

Mouse skin for use in in vitro skin permeation test (Hos: HR-1) was purchased from Hoshino Laboratory Animals, Inc.

### Instruments and apparatuses

Microscope slides and cover slips were purchased from Matsunami Glass Ind., Ltd., and base molds from Thermo Fisher Scientific. A freeze dryer (FDU-1110, EYELA (TOKYO RIKAKIKAI CO., LTD.)), an NMR (ECZ400S), a Polytron homogenizer (Kinematica AG), a dynamic light scattering analyzer (Zetasizer Nano ZS, Malvern Panalytical Ltd.), a fluorescence plate reader (LS-55KG, PerkinElmer, Inc.), a stirrer (RCH-1000, EYELA (TOKYO RIKAKIKAI CO., LTD.)), a hot stirrer (RSH-6DN, REXIM), a confocal laser fluorescence microscope (LSM700, Carl Zeiss AG), a Franz cell (Asahi Glassplant Inc.), a reverse-phase high-performance liquid chromatography (1260 Infinity II), and a cryostat microtome (CM1510, Leica) were used.

First, the cationic surfactant and the anionic surfactants were synthesized in accordance with a protocol in a previous report.

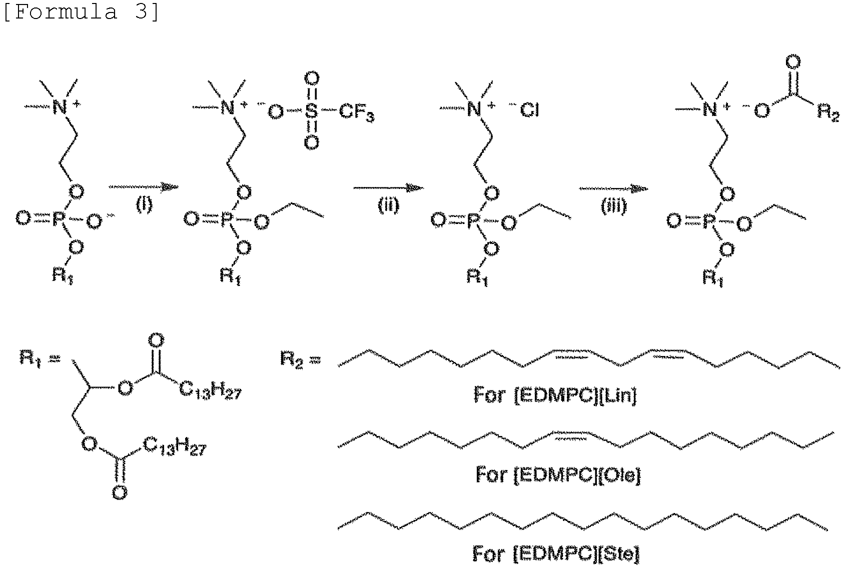

(1) Synthesis of EDMPC-SO₃CF₃: In chloroform solvent, 677.94 mg (1 mmol) of DMPC powder and 129 µL (1 mmol) of ethyl trifluoromethanesulfonate were stirred. This reaction was performed under conditions of a nitrogen flow at 50°C for 12 hours.
(2) Synthesis of EDMPC-Cl: The resulting EDMPC-SO₃CF₃ salt was completely dissolved in chloroform, and then stirred with addition of 5 mL (1 mmol) of 0.2 N hydrochloric acid. The mixture was further washed with Milli-Q water to remove the aqueous phase (containing an excessive portion of hydrochloric acid), and then freeze-dried for 24 hours.
(3) Synthesis of ionic surfactant mixture: The resulting EDMPC-Cl salt was aliquoted into 100-mg portions in three vials, the portions were each dissolved in chloroform, and then stirred with addition of 42 µL of linoleic acid, 42 µL of oleic acid, or 38 mg of stearic acid (mole ratio: 1:1). This reaction was performed under conditions of shading at 50°C for 12 hours. Furthermore, the solutions were freeze-dried for 24 hours to give three ionic surfactant mixtures:
   [EDMPC][Lin],[EDMPC][Ole], and [EDMPC] [Ste].
(4) Identification of ionic surfactant mixtures: The synthesized ionic liquids were subjected to ¹H-NMR measurement using chloroform-d as a solvent.

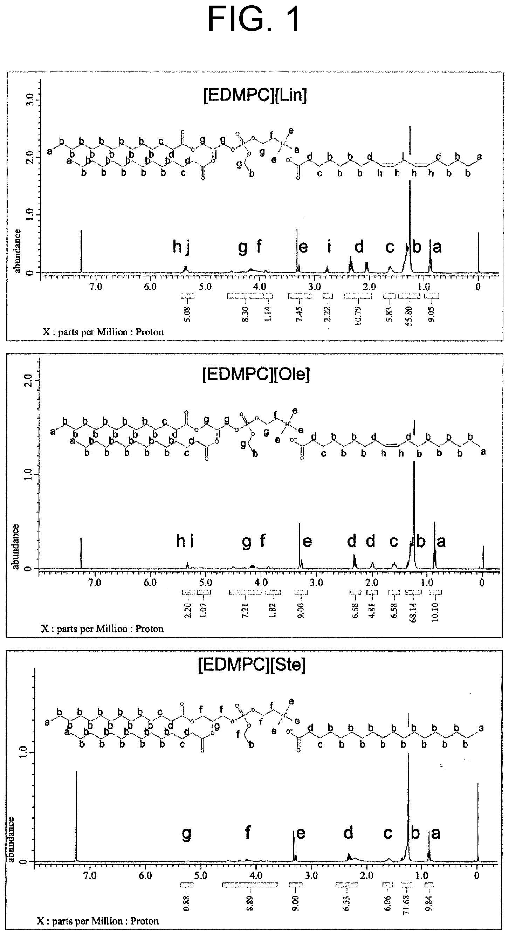

Figure 1 shows the ¹H-NMR spectra of the three ionic surfactant mixtures: [EDMPC] [Lin], [EDMPC] [Ole], and [EDMPC][Ste]. Analysis of the integrated values on the basis of the proton of b found agreement with the theoretical values, confirming that the three ionic surfactants mixtures had been synthesized at a ratio of cation:anion = 1:1.

Each of the ionic surfactant mixtures obtained above, each containing a cationic surfactant and an anionic surfactant, was mixed with a nucleic acid to form an emulsion, which was cryopreserved and then dispersed in an oil phase, giving dispersion solutions each containing: solid complexes each containing a nucleic acid and an ionic surfactant mixture as a dispersoid; and an oil phase as a dispersion medium. The details were as follows.
(1) Formation of emulsions: In 1 mL of Milli-Q water, 1 mg of Trabedersen (ssDNA) was dissolved. Separately, 50 mg of each of the three ionic surfactant mixtures was dissolved in 2 mL of cyclohexane. The aqueous solution and each cyclohexane solution were stirred with a Polytron homogenizer at a high speed of 26,000 rpm for 2 minutes to form an emulsion.
(2) Removal of inner aqueous phase and oil phase: Each of the emulsions was immediately soaked in liquid nitrogen for 20 minutes for freezing, and a freeze dryer was then utilized for a whole day to remove the inner aqueous phase and oil phase; thus, ionic surfactant-nucleic acid solid complexes were produced.
(3) Dispersion in oil: The ionic surfactant-nucleic acid solid complexes were dispersed in 1 mL of IPM, an oily base having high transdermal permeation-accelerating effect, giving dispersion solutions each containing a final concentration of 1 mg/mL of ionic surfactant-nucleic acid solid complexes.

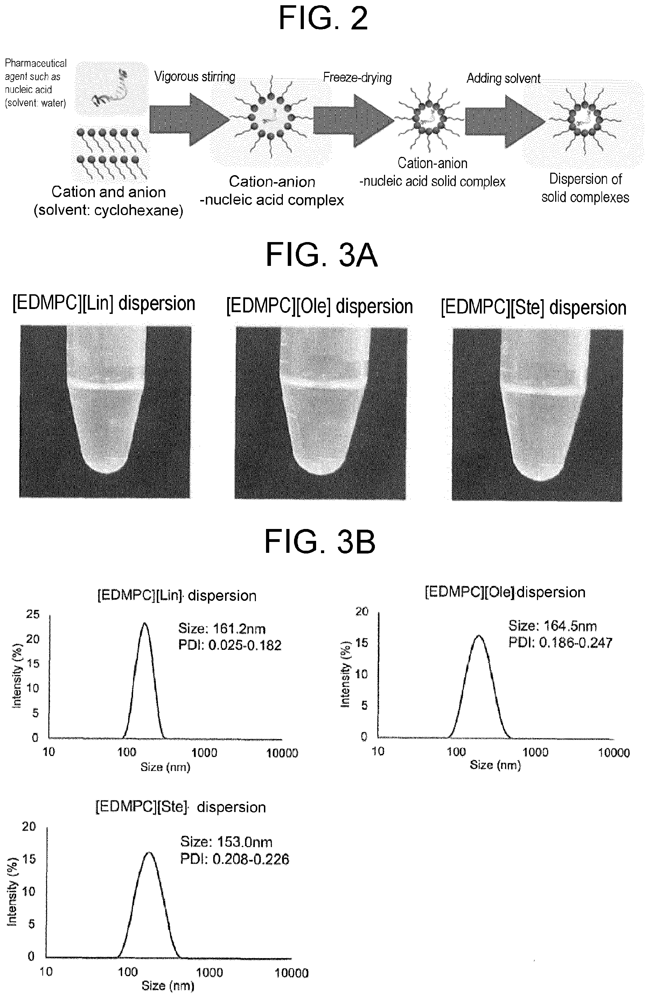

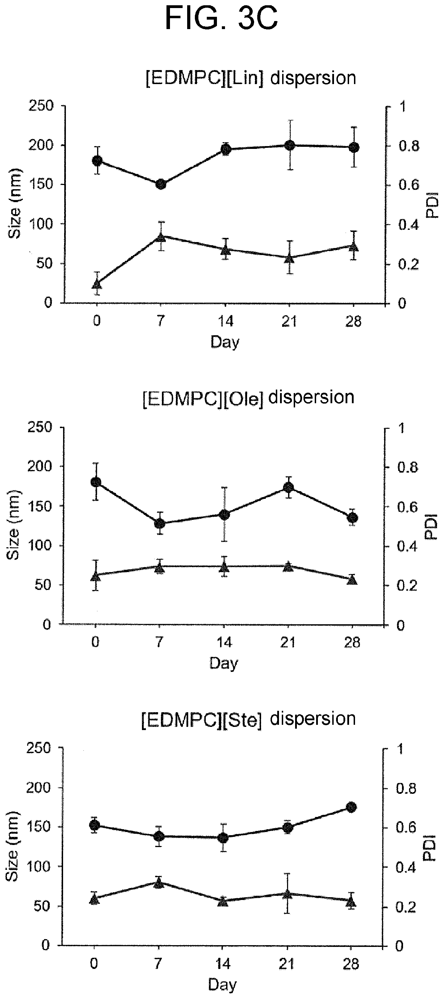

The ionic surfactant-nucleic acid solid complexes thus prepared were subjected to physical properties evaluation by visual observation and dynamic light scattering (DLS) measurement. The DLS measurement was performed under conditions such that each sample was 100-fold diluted with IPM to 0.01 mg/mL. The obtained dispersion solutions each had optical properties with transparency and homogeneity (see Figure 3A). The DLS measurement showed that each contained nanoparticles having hydrodynamic particle sizes of about 100 nm to 200 nm with a single peak (see Figure 3B). The solid complexes were stable until day 28 in the dispersions (Figure 3C).

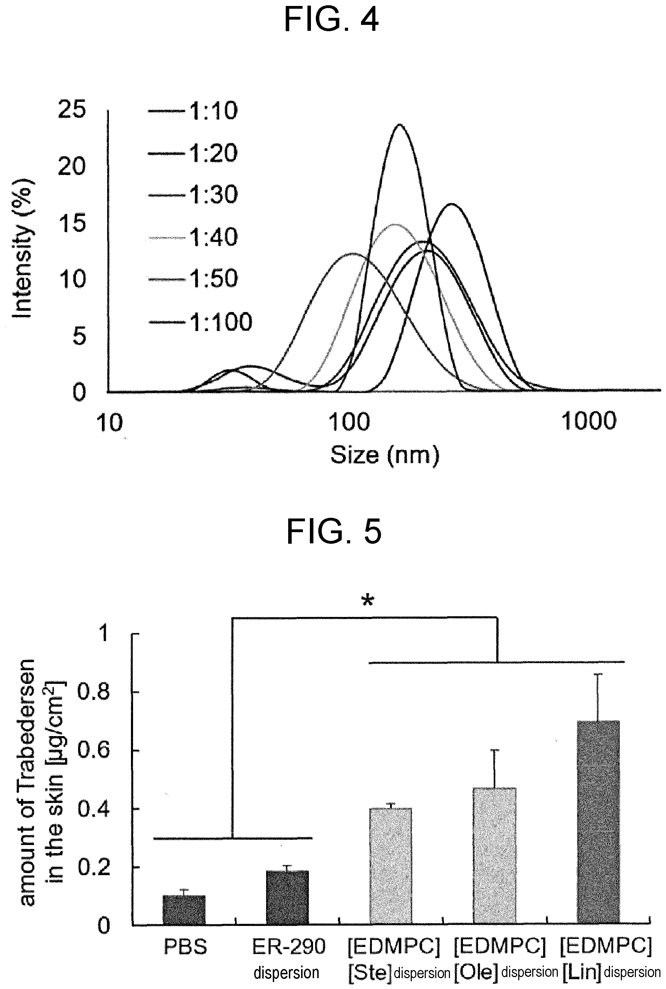

Particle diameter measurement and long-term stability evaluation were carried out with six different mass ratios between a nucleic acid and surfactants: 1:10, 1:20, 1:30, 1:40, 1:50, and 1:100. This examination was performed only for [EDMPC][Lin] among the three surfactant mixtures. The results of the DLS measurement showed that [EDMPC][Lin] exhibited a single peak at any of the mixing ratios, revealing that [EDMPC][Lin] is suitable for preparation of ionic surfactant-nucleic acid solid complexes (see Figure 4).

A dispersion containing conventional surfactant-nucleic acid solid complexes was produced as follows.
(1) Formation of emulsion: In 1 mL of Milli-Q water, 1 mg of Trabedersen (ssDNA) was dissolved. Separately, 50 mg of ER-290 was dissolved in 2 mL of cyclohexane. The aqueous solution and cyclohexane solution were stirred with a Polytron homogenizer at a high speed of 26,000 rpm for 2 minutes to form an emulsion.
(2) Removal of inner aqueous phase and oil phase: The emulsion was immediately soaked in liquid nitrogen for 20 minutes for freezing, and a freeze dryer was then utilized for a whole day to remove the inner aqueous phase and oil phase; thus, ER-290-nucleic acid solid complexes were produced.
(3) Dispersion in oil: The ER-290-nucleic acid solid complexes were dispersed in 1 mL of IPM, an oily base having high transdermal permeation-accelerating effect, giving a dispersion (conventional) having a final concentration of 1 mg/mL.

Transdermal permeation experiment was carried out.
(1) Production of skin pieces: Hairless mouse skin was thawed, and cut into pieces of about 2 cm × 2 cm in size.
(2) Preparation of Franz cell: To the receiver phase of a vertical Franz cell (effective area: 0.785 cm²), 4 mL of PBS was added, mouse skin was set with the SC side facing the doner phase, and 1 mL of PBS was then further added to purge the air in the Franz cell.
(3) Addition of formulation: To each doner phase, 200 µL of a sample was added with stirring with a stirrer and keeping at 32.5°C.
(4) Washing: After the lapse of 6 hours, each mouse skin was taken out, and the horny layer side was washed four times, and the opposite side once with 500 µL of a washing solution (1× TE buffer:acetonitrile = 1:1).
(5) Extraction: The washed skin tissue was shredded into 16 equal parts, which were put in a tube, and soaked in 500 µL of extraction liquid (PBS) and shaken for 24 hours.
(6) Quantitative analysis: The extraction liquid was filtered through a filter, and then subjected to quantitative analysis using an HPLC (see Table 1).

**[Table 1]**

| Table 1: Elution conditions for HPLC | |
|---|---|
| Solvent: | A) 200 mM HCOONH₄ in H₂O, B) 200 mM HCOONH₄ (in H₂O: CH₃CN=50 : 50) |
| Gradient: | A / B = 90 / 10 - 40 min - 60 / 40 - 20 min - 90 / 10 (v/v) |
| Flow rate: | 1.0 mL / min |
| Detection: | UV 260 nm |
| Analytical column: | InertSustain AX-C18, 5 *µ*m, 4.6 mm × 150 mm |

Figure 5 shows the amounts of skin permeation of the samples. The results suggested that the transdermal permeation of the nucleic acid was enhanced in the present dispersions of ionic surfactant-nucleic acid solid complexes as compared with the conventional dispersion. The reason for the enhanced permeation is possibly that the liquid crystal structure of the horny layer was destroyed by the presence of a cationic surfactant and an anionic surfactant. The fact that the obtained solid complexes had a smaller particle size than the conventional solid complexes had is another possible cause for the enhanced skin permeation.

The amount of skin permeation of the [EDMPC] [Lin] solid complexes was the largest among those of the three dispersions. This is probably because the bulkiness of linoleic acid, an anionic fatty acid, with two cis-C=C bonds, resulted in higher ability to disturb horny layer lipid than oleic acid and stearic acid had.

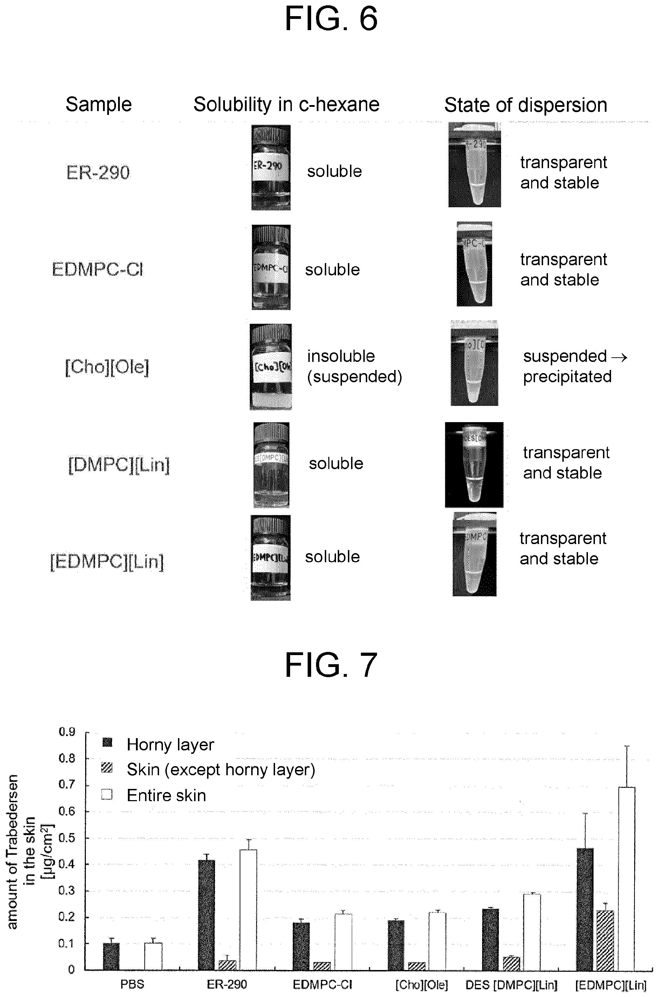

In the above, mixtures each containing an anionic surfactant and a cationic surfactant were used as ionic surfactant mixtures. In the following, dispersion formulations each containing ionic surfactant-nucleic acid solid complexes were produced by using ionic surfactant mixtures each containing an anion and a cation, specifically, [EDMPC] [Cl] with the anion being a Cl⁻ ion, [Cho] [Ole] as another hydrophilic ionic liquid, and DES[DMPC] [Lin] as a simple mixture of DMPC and linoleic acid. The results clearly showed that a stable dispersion containing ionic surfactant-nucleic acid solid complexes was successfully produced for any of the cases (see Figure 6). In the dispersion formulation containing ionic surfactant-nucleic acid solid complexes produced with [Cho][Ole] among those dispersion formulations, the stability of the complexes was found to be relatively low because of the low solubility of the ionic surfactant mixture in cyclohexane. The obtained dispersion formulations were evaluated for permeation into hairless mouse skin in vitro by using a Franz cell as described above. The results showed that the permeation of the nucleic acid into skin was good for any of the cases (see Figure 7). In particular, the dispersion formulation containing ionic surfactant-nucleic acid solid complexes each containing a cationic surfactant and an anionic surfactant ([EDMPC][Lin]) was found to achieve superior cutaneous permeation of the nucleic acid. [DMPC][Lin] was found to have an enhancing effect on the cutaneous permeation of the nucleic acid, but the enhancing effect was only limited. In DMPC, the charges are intramolecularly neutralized, and it was suggested that removal of the negative charge in DMPC to make DMPC cationic is important for enhanced cutaneous permeation. An enhancing effect on cutaneous permeation was also found for choline and Ole, but the enhancing effect was only limited; thus, it was suggested that having a long-chain aliphatic chain is preferred for the cation. The result that a weak cutaneous permeation-enhancing effect was obtained also when propionic acid was used as the fatty acid suggested that having a long-chain fatty acid is preferred also for the anionic lipid. Lipid mixtures such as [EDMPC][Lin] and [EDMPC][Ole] exhibit the characteristics of ionic liquid. In the same test carried out for porcine skin, all of [EDMPC][Lin], [EDMPC][Ole], and [EDMPC][Ste] exhibited higher transdermal permeation of the nucleic acid than the solid complexes produced with ER-290 or PBS exhibited.

The state of cutaneous permeation of a dispersion formulation containing ionic surfactant-nucleic acid solid complexes each containing an anionic surfactant and a cationic surfactant was analyzed under a fluorescence microscope. For this purpose, a dispersion formulation containing ionic surfactant-nucleic acid solid complexes was prepared with fluorescence-labeled ionic surfactants and a fluorescence-labeled nucleic acid.

### Skin permeation-enhancing effect of ionic surfactant mixture containing cationic surfactant and anionic surfactant

The skin permeation-enhancing effect of an ionic surfactant mixture containing a cationic surfactant and an anionic surfactant was examined.
(1) Formation of emulsion: In 1 mL of Milli-Q water, 1 mg of Trabederse (ssDNA) was dissolved. Separately, 50 mg of ER-290 was dissolved in 2 mL of cyclohexane. The aqueous solution and cyclohexane solution were stirred with a Polytron homogenizer at a high speed of 26,000 rpm for 2 minutes to form an emulsion.
(2) Removal of inner aqueous phase and oil phase: The emulsion was immediately soaked in liquid nitrogen for 20 minutes for freezing, and a freeze dryer was then utilized for a whole day to remove the inner aqueous phase and oil phase; thus, ER-290-nucleic acid complexes were produced.
(3) Dispersion in oil: The ER-290-nucleic acid complexes were redispersed in each of IPM containing no ionic surfactant mixture and IPM containing 10 mg of an ionic surfactant mixture, giving dispersions each containing solid complexes with a final nucleic acid concentration of 1 mg/mL.

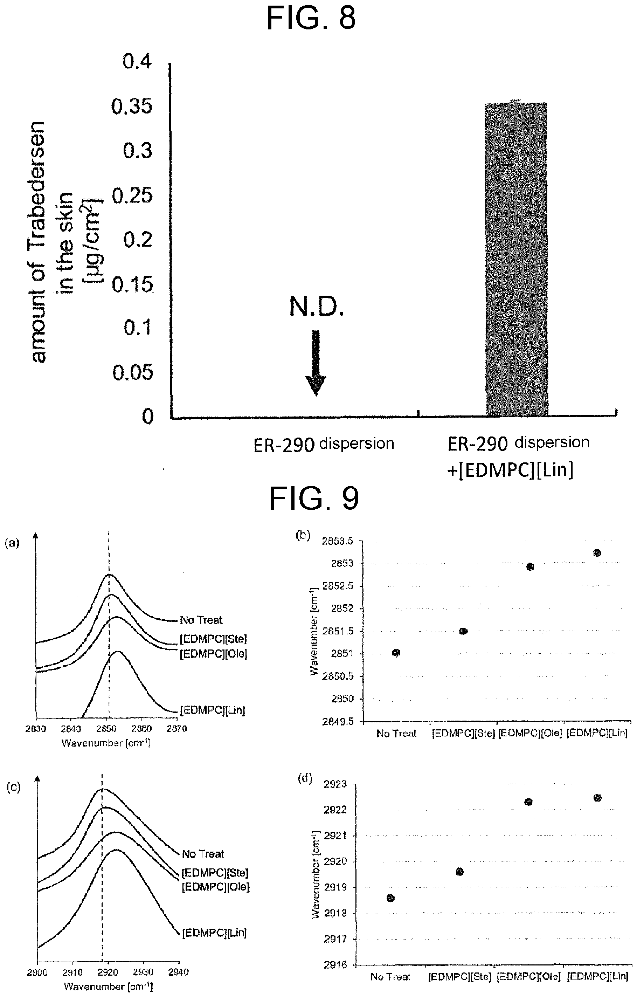

It was clear from the result that addition of an ionic surfactant mixture containing a cationic surfactant and an anionic surfactant to the formulation enhanced the degree of cutaneous permeation of the component of the formulation (see Figure 8).

### Disturbance of horny layer lipid structure by ionic

### surfactant mixture

Cryopreserved YMP skin (back) was thawed at normal temperature. The YMP skin was heated to 60°C with an aluminum incubator for 2 minutes, and the epidermis layer was peeled off with tweezers. With the horny layer side facing up, the epidermis sheet was floated on 0.25% Trypsin solution and left to stand at normal temperature for 24 hours. The living epidermis layer was removed with a cotton swab, and the resultant was dried for 24 hours or more, giving a horny layer sheet. The horny layer sheet was cut into 0.5-cm square pieces, which were wrapped with plastic wrap and stored until use. The three ionic surfactant mixtures [EDMPC][Lin],
[EDMPC][Ole], and [EDMPC][Ste] were directly added to the pieces of the horny layer sheet, which were left to stand at 32.5°C for 1 hour, then washed well with 20% ethanol, and dried for 24 hours. The pieces of the horny layer sheet were subjected to FT-IR measurement, and the peak shifts of CH₂-symmetric stretching vibration (symCH₂) and CH₂-asymmetric stretching vibration (asymCH₂) to appear around 2920 cm⁻¹ and 2850 cm⁻¹, respectively, were observed.

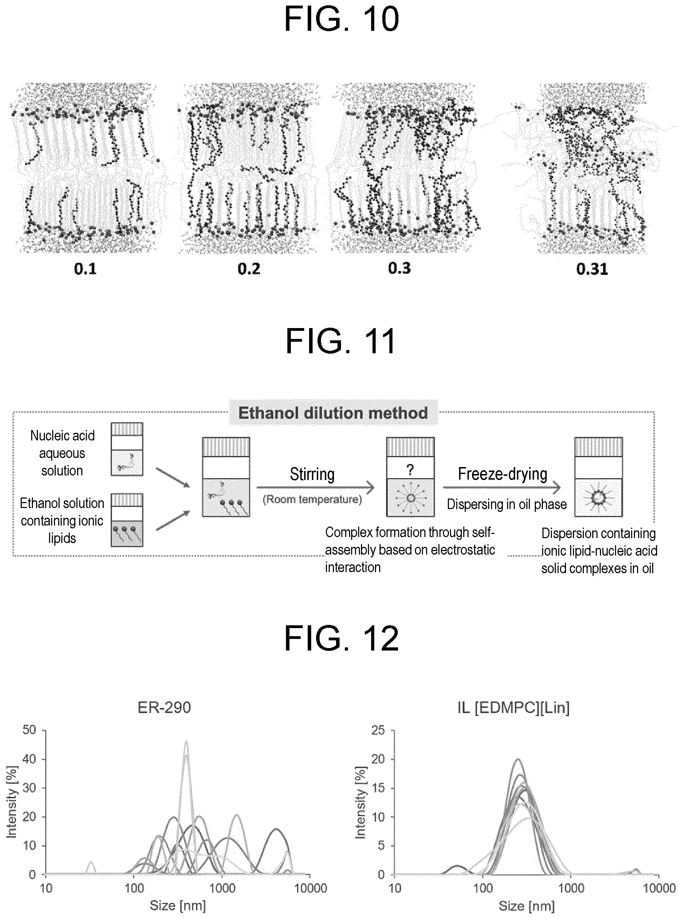

The results showed that the peak shift when [EDMPC][Lin] was applied was the highest, suggesting that the liquid crystal structure of the horny layer lipid was the most disturbed (see Figure 9). The reason why the liquid crystal structure was the most disturbed when linoleic acid was used as an anion is probably that linoleic acid has two cis-C=C bonds and the lipid molecule itself is the most bulky. Akinshina et al. have evaluated the interaction between linoleic acid and intercellular lipid by molecular simulation, and demonstrated that linoleic acid, having cis-C=C bonds, actually entered the lamella structure to destroy the regular structure (see Figure 10) (Anna Akinshina et al., Phys. Chem. Chem. Phys., 18, 17446-17460 (2016)). This result correlates with the result that [EDMPC][Lin]-S/O exhibited the largest amount of permeation among the three ionic surfactant mixtures. This indicates that an ionic surfactant in ionic surfactant-nucleic acid solid complexes is also advantageous in enhancing the cutaneous permeation of a nucleic acid.

### Example 2: Ethanol dilution method

Ionic surfactant-nucleic acid solid complexes were prepared with a process differing from the above.

### Reagents

1,2-Dimyristoyl-sn-glycero-3-phosphatidylcholine (DMPC), linoleic acid, oleic acid, and stearic acid, ethyl trifluoromethanesulfonate, isopropyl myristate, and Span-20 were purchased from Tokyo Chemical Industry Co., Ltd., 5 mol/L hydrochloric acid, chloroform (super-dehydrated), ammonium formate, and ethanol (99.5) from KISHIDA CHEMICAL Co., Ltd., cyclohexane and acetonitrile from FUJIFILM Wako Pure Chemical Corporation, ER-290 and L-195 from Mitsubishi-Kagaku Foods Corporation, 4% paraformaldehyde from MUTO PURE CHEMICALS CO., LTD., Trabedersen and FAM-labeled Trabedersen from Eurofins Genomics K.K., PNA from Fasmac Co., Ltd., and 3-(4,5-dimethyl-2-thiazolyl)-2,5-diphenyl-2H-tetrazolium bromide (MTT) medium from Wako Pure Chemical Industries, Ltd.

### Experimental animals and cells

Mouse skin for use in in vitro skin permeation test (Hos: HR-1) was purchased from Hoshino Laboratory Animals, Inc., B16 melanoma cells from RIKEN, Japan, and the human three-dimensionally cultured epidermis LabCyte EPI-MODEL from Japan Tissue Engineering Co., Ltd.

### Instruments and apparatuses

Microscope slides and cover slips were purchased from Matsunami Glass Ind., Ltd., and base molds from Thermo Fisher Scientific. A freeze dryer (FDU-1110, EYELA (TOKYO RIKAKIKAI CO., LTD.)), a Polytron homogenizer (Kinematica AG), a dynamic light scattering analyzer (Zetasizer Nano ZS, Malvern Panalytical Ltd.), a fluorescence plate reader (LS-55KG, PerkinElmer, Inc.), a hot stirrer (RSH-6DN, REXIM), a confocal laser fluorescence microscope (LSM700, Carl Zeiss AG), a Franz cell (Asahi Glassplant Inc.), a reverse-phase high-performance liquid chromatography (1260 Infinity II), a cryostat microtome (CM1510, Leica), a transmission electron microscope (JEM-2010, JEOL Ltd.), and a small-angle X-ray scattering apparatus (Nanoviewer, Rigaku Corporation) were used.

In Example 1, dispersions were obtained with a technique in which ionic surfactants dissolved in a cyclohexane solution and an aqueous solution containing a nucleic acid were mixed together to form an emulsion, the emulsion is then freeze-dried, and solid complexes finally obtained were dispersed in an oil phase. In the present example, ionic surfactant-nucleic acid complexes were formed with the ethanol dilution method, freeze-drying was then performed, and solid complexes finally obtained were dispersed in an oil phase (Figure 11). The lack of need for the emulsion-forming step in the ethanol dilution method would result in minimized damage in samples. In the present example, whether the method could give particles of solid complexes was examined.

(1) Formation of ionic surfactant-nucleic acid solid complexes: In 5 mL of Milli-Q water, 1 mg of Trabedersen (ssDNA) was dissolved. Separately, 50 mg of an ionic surfactant mixture was dissolved in 1 mL of 99.5% ethanol. The aqueous solution and ethanol solution were mixed together, and gently stirred at room temperature for 6 hours to cause the spontaneous formation of ionic surfactant-nucleic acid solid complexes.
(2) Removal of inner aqueous phase and oil phase: The water/ethanol solution was soaked in liquid nitrogen for 20 minutes for freezing, and a freeze dryer was then utilized for a whole day to remove the inner aqueous phase and oil phase; thus, ionic surfactant-nucleic acid solid complexes were produced.
(3) Dispersion in oil: The ionic surfactant-nucleic acid solid complexes were dispersed in 1 mL of IPM, an oily base having high transdermal permeation-accelerating effect, and adjustment was performed to reach a final nucleic acid concentration of 1 mg/mL, giving a dispersion of solid complexes.

Through that procedure, ionic surfactant-nucleic acid solid complexes were obtained. As surfactants, ER-290 (nonionic surfactant) and a mixture of a cationic surfactant and an anionic surfactant (a mixture of EDMPC and linoleic acid) were used. Analysis of the obtained dispersions by DLS measurement found the particle size distributions as shown in Figure 12. When ER-290, having no charge, was used as a surfactant, the particle size distribution varied among 10 independent experiments; when a mixture of EDMPC and linoleic acid was used, solid complexes in the resulting dispersion exhibited constant particle size distribution.

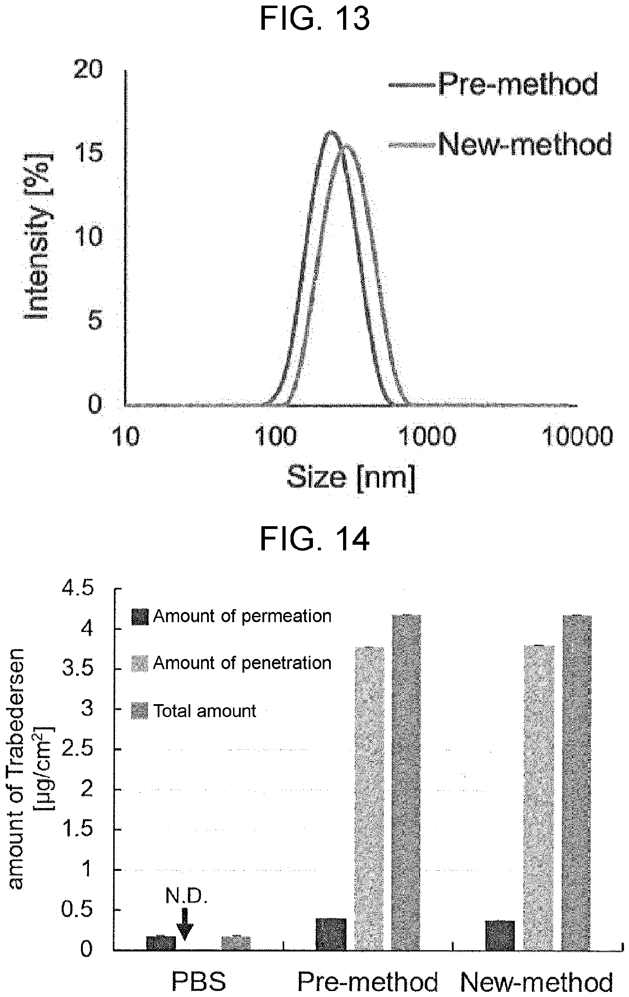

A dispersion containing ionic surfactant-nucleic acid solid complexes (here, the ionic surfactant consisted of a mixture of EDMPC and linoleic acid) prepared as in Example 1 (Pre-method) was prepared, and its skin permeation was compared with that of a dispersion containing ionic surfactant-nucleic acid solid complexes (here, the ionic surfactant consisted of a mixture of EDMPC and linoleic acid) prepared with the ethanol method (New-method). First, the particle size distributions of obtained solid particles were comparable as shown in Figure 13.

Each dispersion was applied to skin, and the amount of cutaneous absorption and the amount of skin penetration were examined. The results showed that the amount of cutaneous absorption and the amount of skin penetration for Pre-method and those for New-method were comparable as demonstrated in Figure 14.

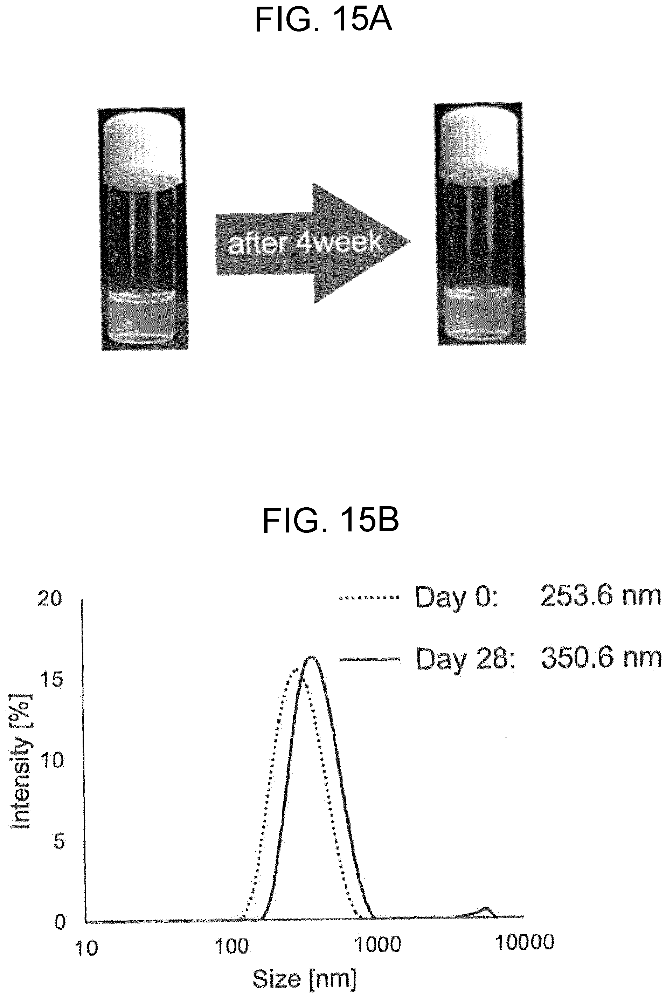

The dispersion obtained with New-method was evaluated for stability for 4 weeks. The particle sizes and PDI were measured by means of DLS immediately after and 4 weeks after the preparation. As shown in Figure 15A, the appearance did not change even after the lapse of 4 weeks. As shown in Figure 15B, the particle size distribution did not change even after the lapse of 4

### weeks.

The composition ratio between the nucleic acid and the ionic surfactant mixture in forming solid complexes was changed. Specifically, ionic surfactant-nucleic acid complexes were newly formed at three mass ratios (N:P ratio with the anion in the lipid ignored) of 1:3 (1:1), 1:15 (1:5), and 1:30 (1:10), and their particle diameter/PDI and skin permeation, and particle diameter/zeta potential in water were compared.

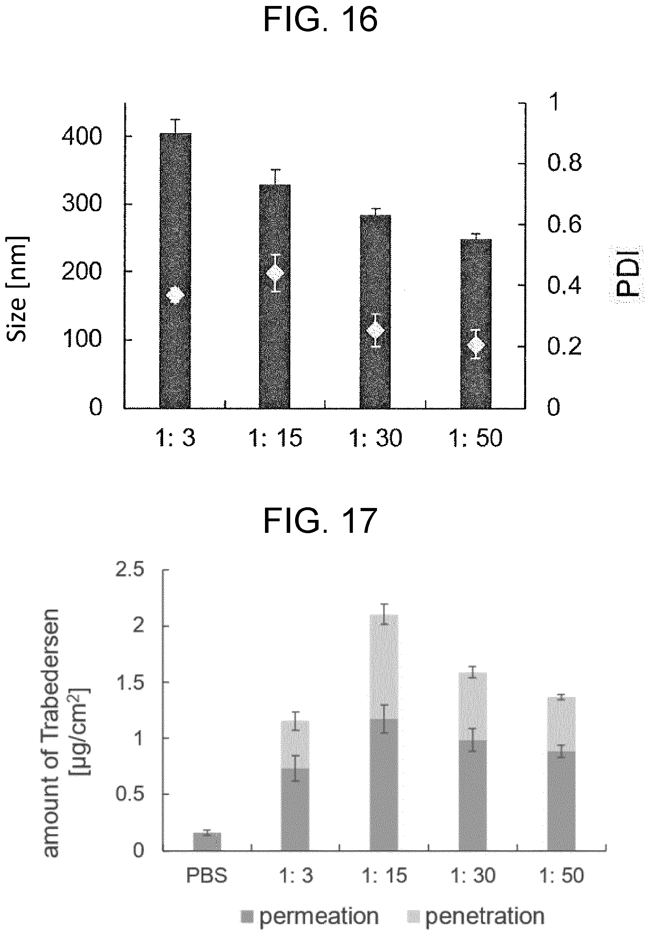

The hydrodynamic particle sizes and PDI in DLS measurement were as shown in Figure 16. As can be seen from Figure 16, a tendency was found such that the larger the amount of the surfactant to that of the nucleic acid, the smaller the particle sizes. Next, the skin permeation and skin penetration of the obtained ionic surfactant-nucleic acid complexes were examined. The results showed that, as shown in Figure 17, the larger the amount of the surfactant to that of the nucleic acid, the more enhanced both the cutaneous permeation and penetration, but that both the cutaneous permeation and penetration rather decreased when the amount of the surfactant exceeded a certain value.

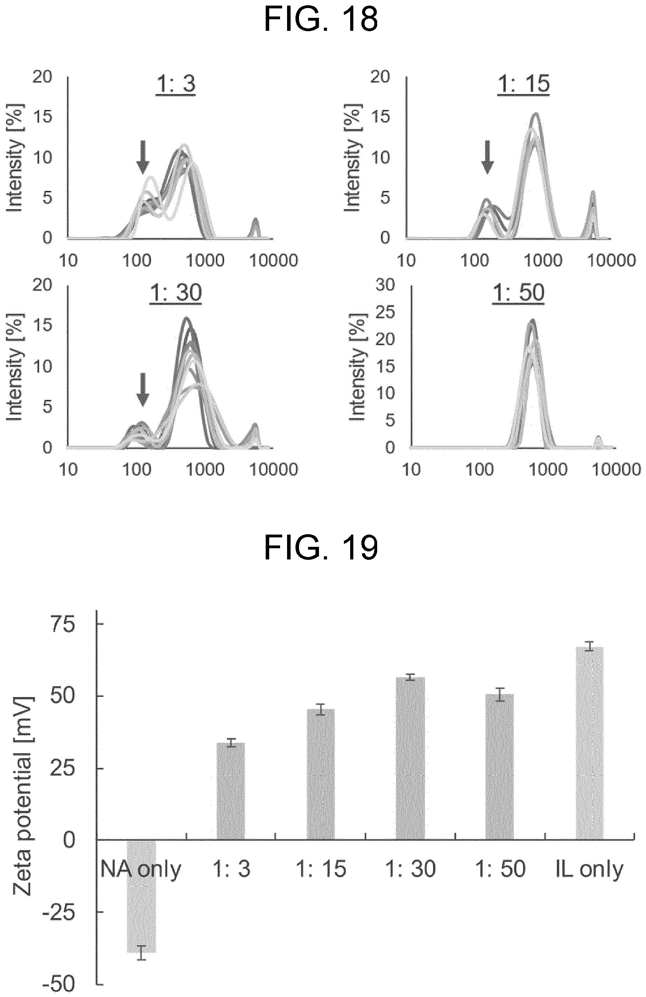

The dispersion produced with the ethanol method was further subjected to analysis by means of DLS (Figure 18) and analysis of zeta potential with a Zetasizer (Figure 19). Positive zeta potential was found in any case, and this suggested the possibility of the presence of a structure in which the nucleic acid was included in the lipid bilayer membrane.

With use of Trabedersen as a nucleic acid, EDMPC as a cationic surfactant, and linoleic acid as an anionic surfactant, a dispersion containing ionic surfactant-nucleic acid solid complexes was prepared with the ethanol dilution method. An aqueous solution containing a nucleic acid and an ethanol solution containing ionic surfactants were mixed together, and the resultant was instantaneously frozen with liquid nitrogen (0 sec), stirred with a vortex mixer for 10 seconds, frozen with liquid nitrogen (10 sec), stirred with a stirrer for 10 minutes or 6 hours, frozen with liquid nitrogen, then freeze-dried, and dispersed in 1 mL of the oily base material IPM.

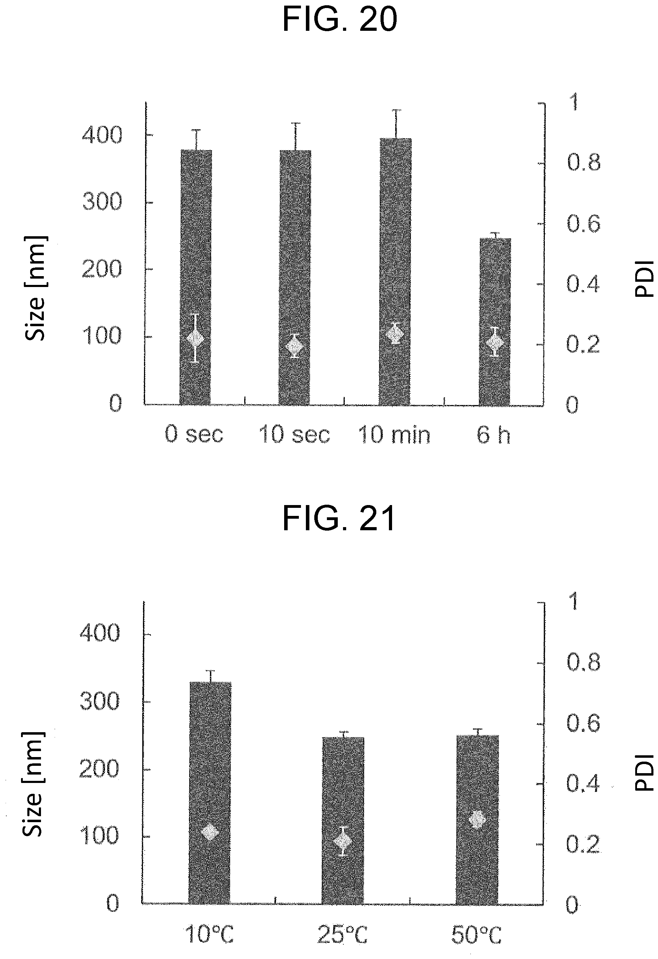

Analysis of particle size distribution (see Figure 20) demonstrated that the ionic surfactants had formed ionic surfactant-nucleic acid solid complexes in an instantaneous manner upon mixing with the nucleic acid.

The relationship between the temperature at which the aqueous solution containing a nucleic acid and the ethanol solution containing ionic surfactants were mixed and the particle size distribution of ionic surfactant-nucleic acid solid complexes obtained was examined. As demonstrated in Figure 21, difference in temperature did not cause change in the particle size distribution of ionic surfactant-nucleic acid solid complexes obtained.

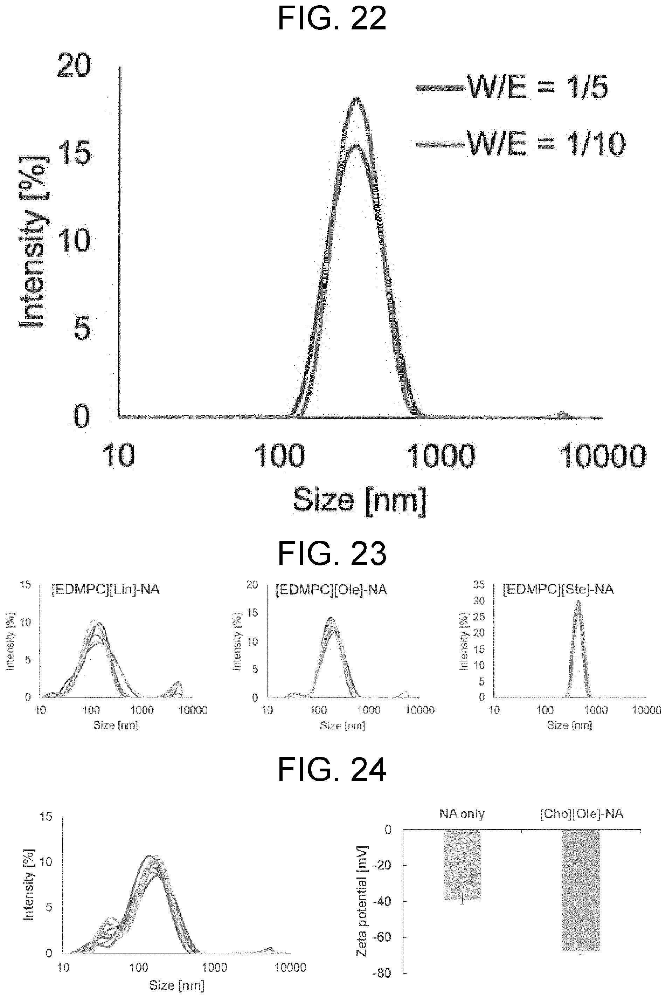

The relationship between the ratio between the aqueous solution and the ethanol solution to be mixed together in the ethanol dilution method and the particle size distribution of ionic surfactant-nucleic acid solid complexes obtained was examined with the ratio set to 1:5 or 1:10. As demonstrated in Figure 22, difference in the mixing ratio between the aqueous solution and ethanol did not cause change in particle size distribution.

With use of EDMPC as a cationic surfactant and linoleic acid, oleic acid, or stearic acid as an anionic surfactant in the ethanol dilution method, the relationship with the particle size distribution of ionic surfactant-nucleic acid solid complexes obtained was examined. As demonstrated in Figure 23, almost single-peaked particle size distribution was exhibited for any of the anionic surfactants used.

With use of choline (choline has no long-chain aliphatic chain) as a cation to be mixed and oleic acid as an anionic surfactant in the ethanol dilution method, the particle size distribution and zeta potential of ionic surfactant-nucleic acid solid complexes obtained were examined. As demonstrated in Figure 24, single-peaked particle size distribution was exhibited, but the zeta potential of the particles was negative. This suggested the possibility that a nucleic acid is not included in particles when a cation having a small number of carbon atoms such as choline is used.

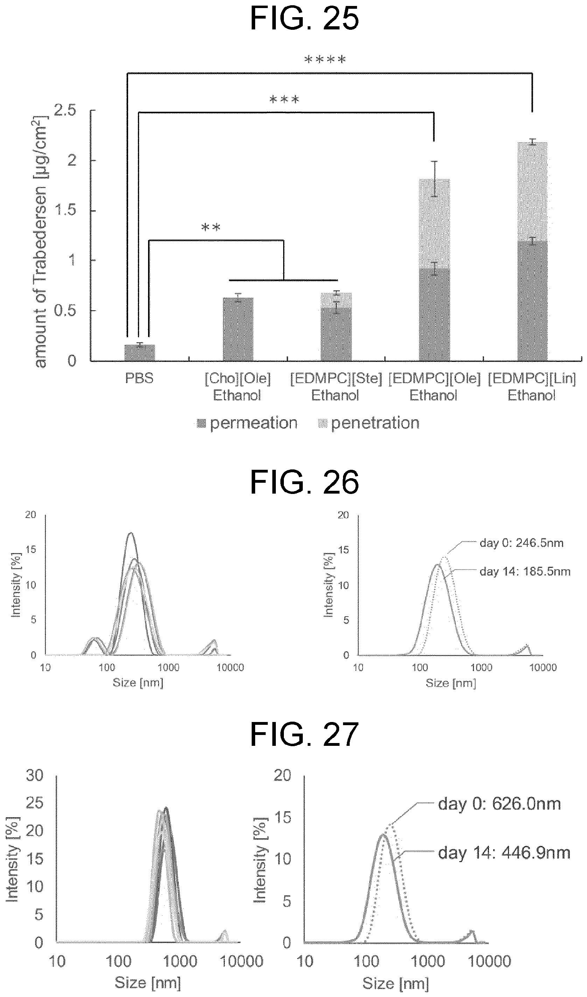

Different ionic surfactant-nucleic acid solid complexes were produced with the ethanol dilution method, and the skin penetration and permeation were evaluated. The results were as shown in Figure 25. As demonstrated in Figure 25, each exhibited enhanced cutaneous permeation. When a cationic surfactant was used as the cation, enhanced skin penetration resulted (see Figure 25).

### Example 3: Peptide nucleic acid

A dispersion containing ionic surfactant-peptide nucleic acid solid complexes was obtained with the ethanol dilution method as described above.
(1) A PNA aqueous solution (nucleic acid 1 mg/Milli-Q water 5 mL) was added to an ethanol solution dissolving [EDMPC] [Lin] therein (5 mg/ethanol 1 mL).
(2) The resultant was gently stirred with a stirrer at room temperature for 6 hours to cause the spontaneous formation of ionic surfactant-nucleic acid complexes.
(3) Thereafter, the inner aqueous phase and oil phase were removed by freeze-drying for 24 hours, and the resultant was redispersed in isopropyl myristate (IPM) to prepare an ionic surfactant-PNA complex formulation (final concentration: 1 mg/mL).

Measurement of the particle size distribution of complexes in the obtained dispersion by means of DLS showed single-peaked particle size distribution (see Figure 26). The dispersion was stable even for 14-day storage (see Figure 26).

### Example 4: Ionic surfactant-protein complexes

Insulin was used as a protein.
(1) An insulin aqueous solution (insulin 1 mg/ water 5 mL was added to an ethanol solution dissolving [EDMPC][Lin] therein (50 mg/ethanol 1 mL).
(2) The resultant was gently stirred with a stirrer at room temperature for 6 hours to cause the spontaneous formation of ionic surfactant-protein complexes.
(3) Thereafter, the inner aqueous phase and oil phase were removed by freeze-drying for 24 hours, and the resultant was redispersed in isopropyl myristate (IPM) to prepare a dispersion containing ionic surfactant-protein solid complexes (final concentration: 1 mg/mL).

Measurement of particle size distribution by DLS measurement showed that the obtained complexes exhibited single-peaked particle size distribution (see Figure 27). The obtained complexes were stable for 14-day storage (see Figure 27).

Ovalbumin was used as a protein.
(1) An OVA aqueous solution (OVA 1 mg/Milli-Q water 5 mL) was added to an ethanol solution dissolving [EDMPC][Lin] therein (5 mg, 10 mg, 25 mg, 50 mg/ethanol 1 mL).
(2) The resultant was gently stirred with a stirrer at room temperature for 6 hours to cause the spontaneous formation of ionic surfactant-protein complexes.
(3) Thereafter, the inner aqueous phase and oil phase were removed by freeze-drying for 24 hours, and the resultant was redispersed in isopropyl myristate (IPM) (with addition of 0, 5, 10, or 25 mg of Span20 as an auxiliary surfactant) to prepare a dispersion containing ionic surfactant-protein solid complexes (final concentration: 1 mg/mL).

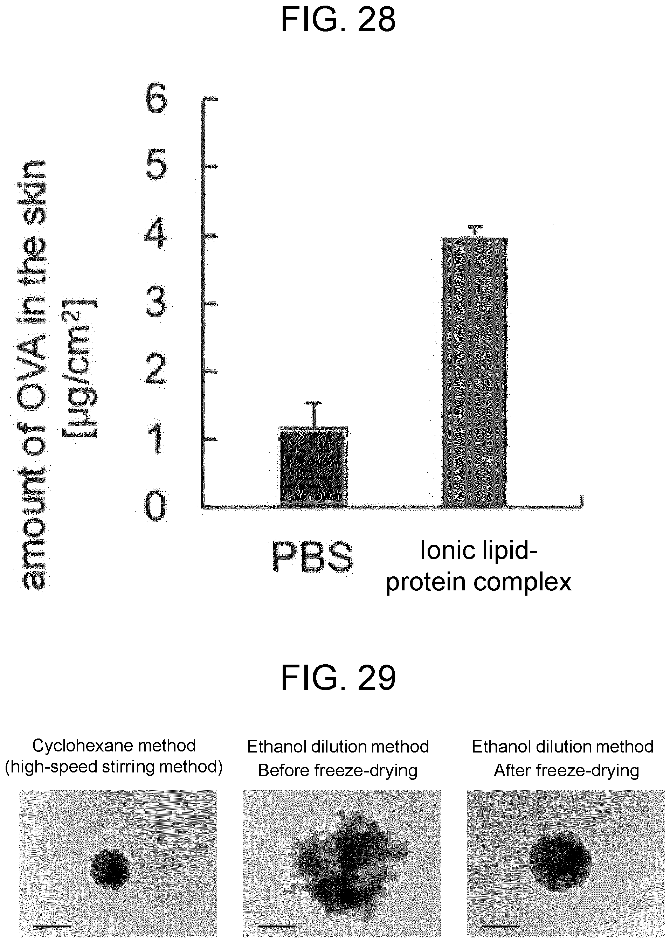

The result showed that the obtained complexes did not exhibit single-peaked particle size distribution. On the other hand, with addition of an ionic surfactant mixture containing Span20 as an auxiliary surfactant, complexes having single-peaked particle size distribution were obtained at a mass ratio of ovalbumin:ionic surfactant mixture:Span20 = 1:25:25. Ovalbumin is a protein of about 45 kDa, which is a protein considerably larger than insulin. Use of an auxiliary surfactant is advantageous for incorporating a large protein in complexes. The cutaneous permeation of the obtained complexes was examined. As demonstrated in Figure 28, the obtained complexes exhibited significantly enhanced cutaneous permeation of ovalbumin.

### Example 5: Structure of ionic surfactant-nucleic acid solid complexes obtained with ethanol dilution method

(1) Formation of ionic surfactant-nucleic acid complexes: In 5 mL of Milli-Q water, 1 mg of Trabedersen (ssDNA) was dissolved. Separately, 50 mg of an ionic surfactant mixture was dissolved in 1 mL of 99.5% ethanol. The aqueous solution and ethanol solution were mixed together, and gently stirred at room temperature for 6 hours to cause the spontaneous formation of ionic surfactant-nucleic acid complexes.
(2) Removal of inner aqueous phase and oil phase: The water/ethanol solution was soaked in liquid nitrogen for 20 minutes for freezing, and a freeze dryer was then utilized for a whole day to remove the inner aqueous phase and oil phase; thus, ionic surfactant-nucleic acid solid complexes were produced.

### Example 6: Electron microscopy for complexes

Samples to be observed were prepared as follows.

### Sample 1: Complexes (in IPM) obtained with high-speed stirring process

(1) Formation of emulsion: In 1 mL of Milli-Q water, 1 mg of Trabedersen (ssDNA) was dissolved. Separately, 50 mg of [EDMPC][Lin] was dissolved in 2 mL of cyclohexane. The aqueous solution and cyclohexane solution were stirred with a Polytron homogenizer at a high speed of 26,000 rpm for 2 minutes to form an emulsion.
(2) Removal of inner aqueous phase and oil phase: The emulsion was immediately soaked in liquid nitrogen for 20 minutes for freezing, and a freeze dryer was then utilized for a whole day to remove the inner aqueous phase and oil phase; thus, ionic surfactant-nucleic acid complexes were produced.
(3) Dispersion in oil: The ionic surfactant-nucleic acid solid complexes were dispersed in 1 mL of IPM, giving a dispersion having a final concentration of 1 mg/mL.

### Sample 2: Complexes (in ethanol aqueous solution) obtained with ethanol dilution method

Formation of ionic surfactant-nucleic acid complexes: In 5 mL of Milli-Q water, 1 mg of Trabedersen (ssDNA) was dissolved. Separately, 50 mg of [EDMPC] [Lin] was dissolved in 1 mL of 99.5% ethanol. The aqueous solution and ethanol solution were mixed together, and gently stirred at room temperature for 6 hours to cause the spontaneous formation of ionic surfactant-nucleic acid complexes.

### Sample 3: Complexes (in IPM) obtained with ethanol dilution method

(1) Formation of ionic surfactant-nucleic acid complexes: In 5 mL of Milli-Q water, 1 mg of Trabedersen (ssDNA) was dissolved. Separately, 5 mg of [EDMPC] [Lin] was dissolved in 1 mL of 99.% ethanol. The aqueous solution and ethanol solution were mixed together, and gently stirred at room temperature for 6 hours to cause the spontaneous formation of ionic surfactant-nucleic acid complexes.
(2) Removal of inner aqueous phase and oil phase: The water/ethanol solution was soaked in liquid nitrogen for 20 minutes for freezing, and a freeze dryer was then utilized for a whole day to remove the inner aqueous phase and oil phase; thus, ionic surfactant-nucleic acid solid complexes were produced.
(3) Dispersion in oil: The ionic surfactant-nucleic acid solid complexes were dispersed in 1 mL of IPM, giving a dispersion having a final concentration of 1 mg/mL.

### TEM observation

Each sample was appropriately diluted with cyclohexane, and cast into a carbon film grid for a scanning transmission electron microscope (STEM). This was dried for 3 hours, and then observed at a magnification of 2000× and an acceleration voltage of 120 kV.

The results were as shown in Figure 29. It was found in the ionic surfactant-nucleic acid solid complexes obtained with the high-speed stirring process (cyclohexane method) that spherical particles of about 10 nm in particle size were aggregating to form an agglomerate of about 200 nm in particle size. It was found in the ionic surfactant-nucleic acid complexes obtained with the ethanol dilution method in the ethanol aqueous solution that disk-like structures of about 10 nm in diameter were aggregating to form a structure of about 600 nm (Figure 29). It was found in the ionic surfactant-nucleic acid solid complexes obtained with the ethanol dilution method that disk-like structures of about 10 nm in particle size were aggregating to form an agglomerate of about 200 nm in particle size (Figure 29).

Ionic surfactant-nucleic acid solid complexes were prepared as follows. Specifically, a freeze-dried product was obtained with the ethanol dilution method as described above in the present example except that FAM-labeled Trabedersen was used as a nucleic acid, and dispersed in IPM, giving a dispersion containing ionic surfactant-nucleic acid solid complexes. Thereafter, the dispersion was further dispersed in Milli-Q water, giving a dispersion solution having a nucleic acid concentration of 1 mg/mL.

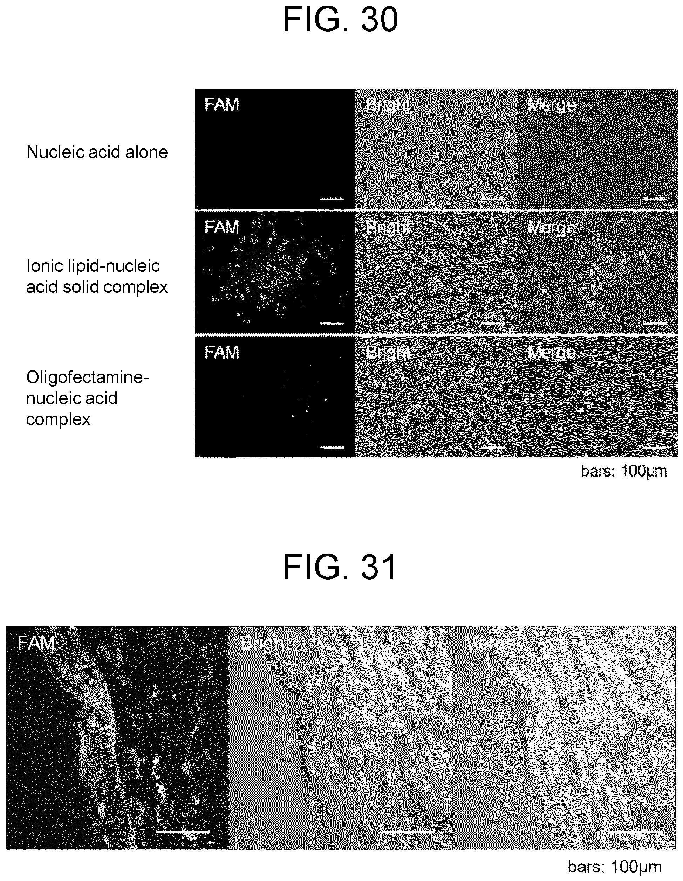

The results found that, as shown in Figure 30, ionic surfactant-nucleic acid solid complexes were efficiently introduced into cells. In view of the production process, the complexes were expected to be in an S/O/W emulsion. The complexes were applied to skin to examine the cutaneous permeation. The skin was isolated and fixed, and a section was then produced to check FAM signals under a fluorescence microscope. The result found that, as shown in Figure 31, the ionic surfactant-nucleic acid solid complexes were incorporated in basal layer cells and dermal cells.

### Example 8: Introduction of mRNA for EGFP into cells and gene expression

Complexes of a cationic surfactant, an anionic surfactant, and an mRNA were formed, and transfection of cells with the mRNA and expression of the mRNA were observed. The mRNA used was for EGFP, and the expression was detected on the basis of fluorescence from EGFP.

A linear double-stranded DNA containing a T7 promoter sequence and encoding green fluorescent protein (EGFP) was used as a template, and EGFP mRNA was synthesized by using a T7 mScript (TM) Standard mRNA production system (CELLSCRIPT) kit. The obtained EGFP mRNA was dissolved in Milli-Q water to 20 ng/µl.

[EDMPC][Lin] was dissolved in 99.5% ethanol to 6 µg/µl. Mixed together were 10 µL of the EGFP mRNA aqueous solution and 1 µL of the [EDMPC][Lin] ethanol solution, and the mixture was lightly vortexed to cause the formation of cationic surfactant-anionic surfactant-mRNA complexes.

B16 melanoma cells were seeded in a multi-well plate (Matsunami Glass Ind., Ltd.) at 1 × 10⁴/well. When the cells reached 70% to 80% confluency on the following day, the medium was exchanged with serum-free medium, and the cationic surfactant-anionic surfactant-mRNA complexes were then added to the medium at a loading of the mRNA of 200 ng/well. The cells were cultured with an incubator at 5% CO₂ and 37°C, and 48 hours after that EGFP-derived green fluorescence was observed with a confocal fluorescence microscope (200×).

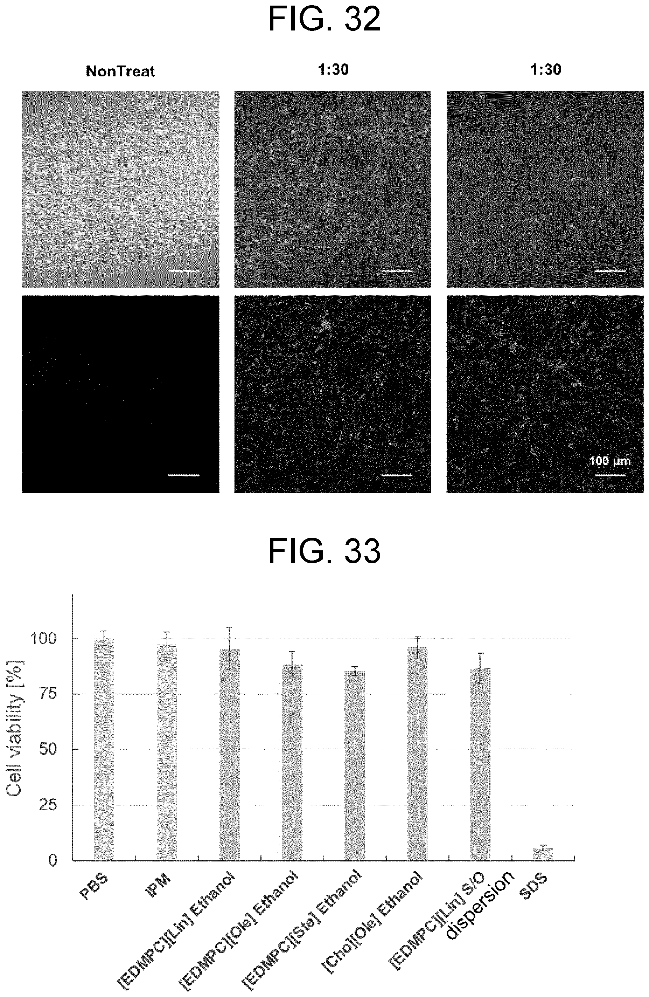

The result was as shown in Figure 32. No fluorescence derived from green fluorescent protein was observed from NonTreat cells, as demonstrated in Figure 32. In the administration system with a cationic surfactant and an anionic surfactant, on the other hand, green fluorescence was observed after 48 hours, indicating that the mRNA was introduced into cells and EGFP was expressed. This revealed that even a long, unstable nucleic acid such as mRNA can be introduced into cells with the nucleic acid kept intact. The ethanol dilution method allows an emulsion-forming step to be omitted, and enables the formation of lipid-nucleic acid complexes only by mixing an aqueous solution containing a nucleic acid and a lower alcohol solution containing surfactants, and the resulting complexes, which are in a dispersed state in an aqueous solution, can be added to culture medium. On coming into contact with cells in the culture medium, the complexes are incorporated into the cells. In this method, lipids having high solubility in alcohols and low solubility in water can be preferably used.

### Example 9: Irritancy to skin

With a human three-dimensionally cultured epidermis model (LabCyte EPI-MODEL (TM)), in vitro skin irritancy test was carried out. The LabCyte EPI-MODEL is a human three-dimensionally cultured epidermis model made by stratified culture of human normal epidermal cells, and has been developed as an alternative material for skin irritancy test with experimental animals. In contrast to animal experiment, the model causes no species difference since human cells are used, has less variation among wells and lots, and is able to provide test results with high reproducibility. Here, with use of a 24-well LabCyte EPI-MODEL24, nucleic acid formulations (ethanol dilution method) with PBS (negative control), IPM (negative control), SDS (positive control), three ionic surfactant mixtures, and [Cho][Ole] as surfactants, and ionic surfactant-nucleic acid solid complexes obtained with [EDMPC][Lin] were evaluated for skin irritancy caused by 15-minute exposure.

### (1) Pre-culture

After warmed in a thermostatic chamber (37°C), assay medium was added into a 24-well plate at 500 µL/well. Culture cups each containing a human epidermis model were taken out with sterilized tweezers, and transferred to the wells each containing the assay medium. Because the culture cups had been on agar medium before that, each culture cup was slowly taken out to allow some air to enter between the culture cup and the agar medium, and if the agar medium was attaching to the outer face of the culture cup, the agar medium was carefully removed with tweezers. The culture cups were transferred to new wells in such a manner that the surface of each epidermis was kept untouched and no bubble was allowed to enter the bottom surface of each culture cup. Thereafter, pre-culture was performed in an incubator overnight (15 to 30 hours).

### (2) Administration and washing of samples

To the center of each human epidermis model, 100 µL of a sample was added with care for 15-minute exposure (n = 3). When a sample did not spread over the whole epidermis, the assay plate was covered with a lid, and the side of the plate was lightly tapped. Next, the samples on the epidermis models were removed, and the culture cups were washed with 100 µL of PBS at 15 times/well (after the final washing, only the outer face of each culture cup was wiped with a sterilized cotton swab to remove PBS).

### (3) Post-culture

Assay medium warmed in a thermostatic chamber (37°C) was added to new wells at 1 mL/well, and the culture cups after washing were then transferred to the new wells. Then, post-culture was performed by culturing in an incubator for 24 hours.

Subsequently, MTT assay was carried out.

Prepared was 5.0 mg/ml 3-(4,5-dimethylthiazole-2-thiazole)-2,5-diphenyl-2H-tetrazolium bromide (MTT)/DMEM medium (MTT medium), and filter-sterilized. This MTT medium was added to new wells at 500 µL/well, and the culture cups were transferred thereto. Thereafter, the resultant was reacted in an incubator for 3 hours.

### Extraction of MTT reaction product (formazan)

After the MTT reaction, the human epidermal tissues in the culture cups were taken out with tweezers, and each soaked in 300 µL of isopropanol in a cold and dark place overnight (15 hours or more) to extract dyes.

### Absorbance measurement and calculation of viable cell counts

The absorbances at 570 and 650 nm of each extract solution were measured with a plate reader. Then, viable cell rates, with the cell viability of a negative control (PBS) set to 100%, were calculated from the following expressions: Measurement = [Absorbance (570 nm) of specimen - Absorbance (650 nm) of specimen] - Absorbance (570 nm) of blank - Absorbance (650 nm) of blank] Viable cell rate (%) = (Mean of measurements for test substance) / (Mean of measurements for negative control) × 100.

As shown in Figure 33, all the components had no significant influence on cell viability, thus expected to be nonirritating to skin.

### Example 10: Transdermal delivery of nucleic acid to tumor, skin, and liver

A formulation targeting SNORA23 was prepared. To mice, 3 × 10⁶ ES2 ovarian carcinoma cells were subcutaneously transplanted, and the formation of subcutaneous tumor was checked 1 week after that. To the top (1 cm × 1 cm) of a tumor or into the periphery of a tumor, 50 µL of hybrid particles containing 250 µg of ASO targeting SNORA23 was applied or subcutaneously injected. For the transdermal administration group, protective tape was wound once around the trunk including the administration site.

The ASO had the following sequence:
5'-GAAacctatgmCAmCa-3' (SEQ ID NO: 2)
wherein lower case letters represent DNA nucleotides, capital letters represent LNA-modified nucleotides, and mC represents LNA-modified methylcytosine; and all the phosphate groups are phosphorothioate-modified.

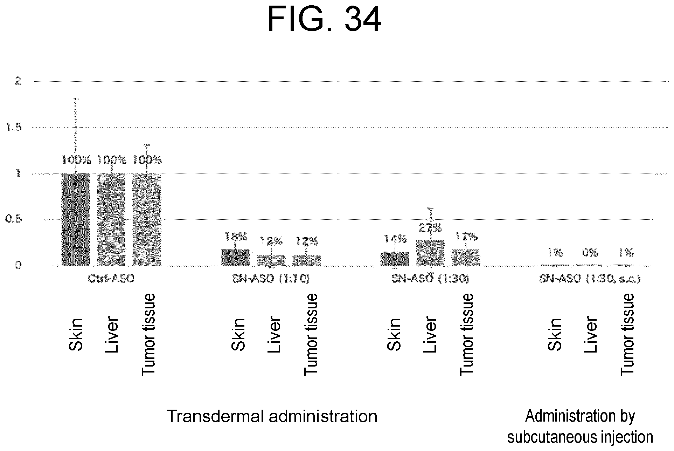

Two days after the administration, the skin, subcutaneous tumor, and liver were isolated, and total RNA was extracted. The level of targeted RNA (SNORA23) was quantified, and the presence or absence of reduction in the expression level was examined by an RT-PCR method. The results were as shown in Figure 34. Reduction of the expression level of the target RNA was induced in any of the transdermal administration and subcutaneous administration groups, as demonstrated in Figure 34. The knock-down effect in the liver suggests the migration of ASO into blood irrespective of the route of administration.

## Claims

1. A composition for use in formation of a complex with a pharmaceutical ingredient, the composition comprising a mixture of a cation and an anion.

2. The composition according to claim 1, wherein the cation is a cationic lipid, and the anion is a fatty acid.

3. The composition according to claim 1 or 2, comprising no pharmaceutical active ingredient.

4. The composition according to claim 1 or 2, further comprising a pharmaceutical active ingredient to be transdermally administered.

5. A composition comprising:
a population of solid complexes each comprising a cation, an anion, and a nucleic acid; and
an oil phase, wherein
the population of the solid complexes is dispersed in the oil phase.

6. The composition according to claim 5, wherein the population of the solid complexes comprises a solid complex of about 5 nm to about 50 nm.

7. The composition according to claim 6, wherein the population of the solid complexes comprises a structure comprising a plurality of aggregates of solid complexes of about 5 nm to about 50 nm.

8. The composition according to any one of claims 5 to 7, for use in transdermal administration of the nucleic acid.

9. The composition according to any one of claims 5 to 8, wherein the cation is a cationic surfactant, and the anion is an anionic surfactant.

10. The composition according to any one of claims 5 to 9, wherein the cation and the anion are comprised at an electric charge ratio of 1:0.8 to 1:1.2.

11. The composition according to any one of claims 5 to 10, wherein the cation and the anion are capable of forming an ionic liquid at least when the cation and the anion are mixed together at an electric charge ratio of 1:1 in a composition consisting only of the cation and the anion.

12. An aqueous composition comprising a complex comprising a cation, an anion, and a nucleic acid, wherein
the composition allows transfection with the nucleic acid from an exterior of a cell to an interior of the cell and thereby enables production of a viable cell comprising the nucleic acid therein.

13. A method for producing the composition according to any one of claims 5 to 11, the method comprising:
mixing an aqueous solution comprising a nucleic acid and a lower alcohol solution comprising a mixture comprising a cation and an anion to provide a complex comprising the nucleic acid and the cation and anion.

14. The method according to claim 13, further comprising freeze-drying the complex provided.

15. The method according to claim 12 or 13, comprising dispersing a freeze-dried complex in an oil phase.

16. The composition according to claim 12, for use in transfection with a nucleic acid from an exterior of a cell to an interior of the cell.

17. A composition for use in attenuating skin barrier (or enhancing skin permeation) in a subject, the composition comprising an anionic lipid.

18. The composition according to claim 17, wherein the anionic lipid is a fatty acid.

19. The composition according to claim 17 or 18, wherein the anionic lipid is a long-chain fatty acid.

20. The composition according to any one of claims 17 to 19, wherein the anionic lipid is a long-chain unsaturated fatty acid.
